# FASCICULE DE BREVET EUROPEEN

(11) **EP 2 084 166 B1**
(45) Date de publication et mention de la délivrance du brevet: **22.11.2017**
(21) Numéro de dépôt: 07823292.3
(22) Date de dépôt: 17.07.2007
(51) Int. Cl.: C07C 317/14, C07C 323/67

(54) **PROCÉDÉ DE PRÉPARATION DE SULFONYLIMIDURES AROMATIQUES**
HERSTELLUNGSVERFAHREN VON AROMATISCHEN SULFONYLIMIDEN
PREPARATION PROCESS OF AROMATIC SULFONYLIMIDES

(30) Priorité: 17.07.2006 FR 0606469
(43) Date de publication de la demande: 05.08.2009
(73) Titulaire: Institut National Polytechnique de Grenoble, 38031 Grenoble (FR); Eras-Labo, 38330 Saint-nazaire-les-eymes (FR); Université Claude Bernard Lyon 1, 69100 Villeurbanne (FR)
(72) Inventeur: SANCHEZ, Jean-Yves, 38330 Saint Ismier (FR); LANGLOIS, Bernard, 69006 Lyon (FR); MEDEBIELLE, Maurice, 69300 Caluire Et Cuire (FR); TOULGOAT, Fabien, 69100 Villeurbanne (FR); ALLOIN, Fannie, 38220 Vizille (FR); PAILLARD, Elie, 67000 Strasbourg (FR); IOJOIU, Cristina, 38000 Grenoble (FR)
(74) Mandataire: Goulard, Sophie
(86) Numéro de dépôt international: PCT/FR2007/001225
(87) Numéro de publication internationale: WO 2008/009814

(56) Documents cités:
- EP-A- 0 364 340
- EP-A- 1 635 218
- WO-A-2005/113491
- US-A- 5 256 821
- US-A- 5 514 493
- A. BLASCHETTE ET AL.: "Polysulfonylamine, XXXVI. Trimethylsilyl-dimesylamin: Darstellung, NMR-spektroskopische Charakterisierung und Reaktionsfähigkeit als Silylierungsreagens" Z. NATURFORSCHUNG, vol. 47b, 1992, pages 1693-1700, XP009083502

## Description

La présente invention concerne un procédé de préparation de sulfonylimidures.

Il est connu d'utiliser des bis(fluorosulfonyl)imidures de lithium comme sels pour l'électrolyte dans divers dispositifs électrochimiques, notamment dans les batteries. Les composés les plus représentatifs sont des composés du type (R_{F}SO₂)₂NM dans lesquels R_{F} est un groupe perfluoroalkyle et M est cation de métal alcalin ou un cation organique. Ces composés présentent des propriétés relativement bonnes, mais cependant insuffisantes.

Il est connu de préparer des sulfonylimidures [RSO₂NSO₂R']M par réaction d'un fluorure de sulfonyle RSO₂F avec un sulfonamide R'SO₂NH₂. Cependant, la réaction de condensation est en compétition avec une hydrolyse et le sulfonylimidure est obtenu avec un faible rendement. En outre, il est très difficile de modifier le groupe R_{f} qui a un caractère électroattracteur (en vue de lui conférer des propriétés additionnelles, permettant par exemple un greffage ou une polymérisation) sans dégrader le groupe sulfonimide. A titre d'exemple, EP 1 635 218 A2 décrit la préparation de sulfonylimides R¹SO₂NHSO₂₋A-SO₂-B-R et de sels de sulfonium et d'iodonium correspondants destinés à être utilisés dans des compositions photosensibles.

Il est également connu de EP 0 364 340 A1 et US 5,256,821 de préparer des sulfonylimidures [RSO₂NSO₂R]M par réaction d'un composé silazane M(((R₂)₃Si)₂N)_{y} avec au moins une composante halogénure de sulfonyle RSO₂F ou RSO₂Cl, avec R, identiques ou différents, et représentant chacun un radical organique monovalent hydrocarbyle en C₁ à C₁₂ et plus particulièrement un radical perfluorohydrocarbyle.

La présente invention a pour but de proposer un nouveau procédé de préparation de sulfonylimidures fonctionnalisés.

En conséquence, la présente invention a pour objet un procédé de préparation des sulfonylimidure.

Le procédé de l'invention est destiné à la préparation de sulfonylimidures répondant à la formule [R-SO₂-N-SO₂R']ᵣM (I), et il consiste à préparer un sulfonimide N-substitué (II) RSO₂N(R")SO₂R' à partir de RSO₂F (III), et à remplacer le groupe R" par une réaction de substitution nucléophile pour obtenir le sulfonylimidure (I), R" étant un groupe benzyle, allyle ou triméthylsilyle, étant entendu que, dans les formule (I), (II) et (III) :
- R représente un groupe répondant à la formule Ar-Z-L- ;
- R' représente un groupe perfluoroalkyle linéaire ou ramifié, de préférence un groupe CF₃ ou C₂F₅, ou un groupe identique au groupe Ar-Z-L- qui constitue R ;
- Z représente un groupe sulfure, sulfinyle, ou sulfonyle, ;
- L représente un groupe de formule -(CF₂)ₙ-CFR^{f}- ;
- n est 0 ou 1 ;
- R^{f} représente F ou un groupe perfluoroalkyle ayant de 1 à 8 atomes de carbone ;
- Ar représente un groupement aromatique choisi dans le groupe constitué par les groupements aromatiques monocycliques ; les groupements aromatiques polycycliques ayant des noyaux condensés ou non condensés ; et les groupements aromatiques hétérocycliques, bicycliques à cycles condensés ou non, ou monocycliques ;
- M est un cation de valence r, choisi parmi les cations de métal alcalin, de métal alcalino-terreux, ou de métal trivalent ou tétravalent, et les cations organiques choisi parmi les ions ammonium, phosphonium, imidazolium, guanidinium, pipéridinium, pyrolidinium, pyridinium, ou quinolinium.

Le procédé de préparation du sulfonylimide N-substitué RSO₂N(R")SO₂R' (II) à partir du fluorure de sulfonyle RSO₂F (III) dépend de la nature du groupe R'.

Lorsque le groupement R' est un groupe identique au groupe Ar-Z-L qui constitue R, le composé (II) est obtenu par réaction du composé R-SO₂F avec un sel d'hexaméthyldisilazane (Me₃Si)₂N⁻A⁺ (A étant un cation de métal alcalin ou un ion ammonium quaternaire) et il répond à la formule (IIa).
La réaction libère le fluorure AF qui génère immédiatement la substitution nucléophile sur le composé II avec remplacement du groupe triméthylsilyle par le cation A⁺, avec production du sulfonimidure symétrique [(Ar-Z-L-SO₂)N]₂A.

Lorsque R' est un groupe perfluoroalkyle, le composé (II) répond à la formule RSO₂N(R")SO₂R" (IIb), R" étant un groupe benzyle ou un groupe allyle, et il est obtenu par l'intermédiaire d'un composé RSO₂NH(R") (II'b). Le procédé de préparation du composé (IIb) comprend la préparation du composé (II'b) par réaction de R-SO₂F (III) avec la benzylamine ou l'allylamine en excès, puis la neutralisation par HCl et l'isolement de (II'b), puis la réaction du composé (II'b) avec l'anhydride (R'SO₂)₂O, en présence d'une amine tertiaire, par exemple la diisopropyléthylamine (DIEA). La réaction peut être effectuée dans le dichloroéthane. Le processus global peut être représenté par le schéma réactionnel suivant :

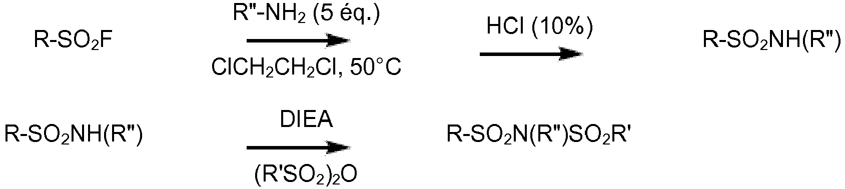

Le réactif qui réagit, de préférence en présence d'un alcool (par exemple l'éthanol), avec un sulfonylimide N-substitué (IIb) pour la substitution nucléophile lors de la 2^{ème} étape peut être choisi parmi :
- les hydroxydes et les halogénures de métal alcalin ou de métal alcalino-terreux pour obtenir des sulfonimidures métalliques ;
- les hydroxydes et les halogénures d'ion ammonium quaternaire, pour obtenir les sulfonimidures d'ions ammonium quaternaire ;
- les alcoolates de métal alcalin ou de métal alcalino-terreux, pour obtenir des sulfonimidures métalliques ;
- les alcoolates d'ammonium quaternaire, pour obtenir les sulfonimidures d'ions ammonium quaternaire ;
- les alcoolates d'ammonium quaternaire, pour obtenir les sulfonimidures d'ions ammonium quaternaire ;
- les amidures de métal alcalin ou de métal alcalino-terreux, pour obtenir des sulfonimidures métalliques ;
- les amidures d'ammonium quaternaire, pour obtenir les sulfonimidures d'ions ammonium quaternaire ;
- les amines secondaires, pour obtenir des sulfonimidures d'ion ammonium tertiaire dont l'un des substituants est un groupe benzyle ;
   - les amines tertiaires, pour obtenir des sulfonimidures d'ion ammonium quaternaire dont l'un des substituants est un groupe benzyle.Dans l'art antérieur, les sulfonimidures dissymétriques sont le plus souvent préparés par réaction d'un fluorure de sulfonyle avec un sulfonamide R_{F}SO₂NH₂ ou son dérivé silylé R_{F}SO₂N (Na) SiMe₃ selon les réactions suivantes :

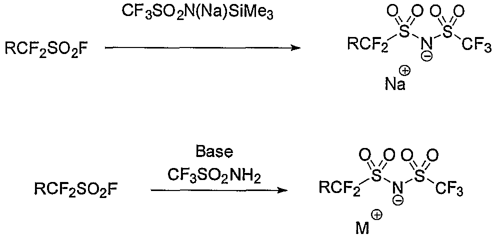

Cependant, les réactifs utilisés sont hygroscopiques et mauvais réactifs nucléophiles. Ainsi, si la réaction n'est pas effectuée dans des conditions strictement anhydres, une réaction d'hydrolyse est en compétition avec la réaction de formation des sulfonylimidures avec formation de sulfonate, qui est très difficile à éliminer.

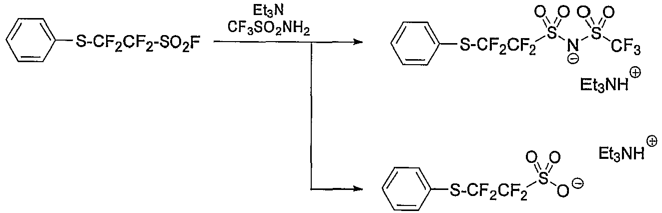

Le procédé de la présente invention, qui associe une réaction préliminaire de couplage d'un fluorure de sulfonyle avec la benzylamine (qui est un meilleur nucléophile que R_{f}SO₂NH₂), permet une sélectivité nettement plus grande que le couplage classique avec R_{f}SO₂NH₂. La réaction d'hydrolyse qui produit le sulfonate de benzylammonium devient minoritaire dans ces conditions, et la réaction largement majoritaire correspond au schéma réactionnel suivant :

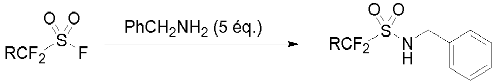

De plus, la purification des N-benzylsulfonamides est beaucoup plus aisée que celle des sulfonylimidures. En outre, les transformations suivantes (formation des N-Benzylsulfonimide, puis déprotection dans l'alcool) sont très sélectives et les sous-produits générés sont très facilement éliminés.

Lorsque le cation du sulfonylimidure est un cation organique, il peut porter un ou plusieurs substituant(s) choisis indépendamment les uns des autres parmi :
o l'hydrogène ;
o les groupes alkyle ;
o les groupements aromatiques monocycliques ; les groupements aromatiques polycycliques à noyaux condensés ou non condensés ; les groupements aromatiques hétérocycliques dans lesquels l'hétéroatome est un atome d'azote, lesdits groupements hétérocycliques étant polycycliques à noyaux condensés ou non condensés, ou monocycliques.

Comme exemples particuliers de cations organiques, on peut citer les cations dialkylammonium et trialkylammonium, par exemple (C₂H₅)₂NH²+, (C₂H₅)₃NH⁺, (C₄H₉)₂NH₂⁺ et (C₄H₉)₃NH⁺.

Un groupe aromatique Ar peut faire partie d'une unité récurrente d'une chaîne polymère.

Le groupe Ar peut porter un ou plusieurs substituants choisis parmi :
- les atomes d'halogène, Cl-CH₂-, et les groupes Q¹-O-CH₂- dans lesquels Q¹ est H, un groupe alkyle ou un groupe acyle ;
- Un groupement hydroxyle, les groupes éther Q²-O-, ester carboxylique Q²C(O)O- et sulfonate Q²-SO₂-O-, Q² représentant un groupement alkyle ou un groupement aryle, [par exemple l'éther de benzyle PhCH₂O-, l'éther de trityle Ph₃CO-, l'éther de méthyle CH₃O-, l'ester de benzoyle PhC(O)O- et l'ester d'acétyle CH₃C(O)O-] ;
- les groupements aliphatiques ou aromatiques possédant une insaturation éthylénique [provenant par exemple d'un motif vinyle (CH=CH₂-), d'un motif allyle (CH₂=CH-CH₂-) ou d'un motif acryloyloxy (CH₂=CH-C(O)-O-)], éventuellement substitué ;
- les groupements amino -N(Q³) (Q⁴)- dans lesquels Q³ et Q⁴ représentent chacun indépendamment de l'autre H, un groupe alkyle, un groupe aryle, un groupe arylalkyle ou un groupe acyle, [par exemple CH₃C(O)NH- ou PhCH₂NH-)] ;
- les groupes trialkylsilyle ;
- les groupes oxirane ;
- les groupements électroattracteurs tels que les groupements perfluoroalkyle, les groupes alkylsulfonyle ou arylsulfonyle, les groupes halogénure de sulfonyle, les groupes ester, nitrile, carbonate cyclique ou nitro.

Une 1^{ère} famille de sulfonylimidures comprend les composés répondent à la formule (I) dans laquelle M est un cation monovalent, et R' est Ar-Z-L. Ces composés symétriques répondent aux formules:
- (Ar-S-L-SO₂)₂N⁻M⁺ (Iaₛ) ;
- (Ar-SO-L-SO₂)2N⁻M⁺ (Ia_{SO}) ;
- (Ar-SO₂L-SO₂)₂N⁻M⁺ (Ia_{SO2}) .

Une 2^{ème} famille de sulfonylimidures comprend les composés qui répondent à la formule (I) dans laquelle R' est un groupe perfluoroalkyle CₚF₂ₚ₊₁, p étant de préférence de 1 à 8, plus particulièrement de 1 à 2, à savoir [Ar-Z-L-SO₂-N-SO₂ CₚF₂ₚ+1]ᵣM (Ib).

Une 3^{ème} famille de sulfonylimidures comprend les composés qui répondent à la formule (I) dans laquelle L est CF₂ ou (CF₂)₂, à savoir [Ar-Z-(CF₂)₂-SO₂-N-SO₂R']ᵣM (Ic) et [Ar-Z-CF₂-SO₂-N-SO₂R']ᵣM (Ic') .

Une 4^{ème} famille de sulfonylimidures comprend les composés (Id) qui répondent à la formule (I) dans laquelle Ar fait partie d'une unité récurrente d'une chaîne polymère.

Une 5^{ème} famille de sulfonylimidures comprend les composés qui répondent à la formule (I) dans laquelle M est un cation monovalent et qui répondent à la formule (Ar-Z-L-SO₂)N(SO₂-R')⁻M⁺ (Ie). Parmi les composés Ie, on peut mentionner en particulier :
∘ les sulfonylimidures de lithium Ie_{Li} ;
∘ les sulfonylimidures d'ammonium Ieₐₘₘ dans lesquels l'ion est un ammonium quaternaire ;
∘ les sulfonylimidures d'ammonium Ieₐₕ dans lesquels l'ion ammonium porte au moins un atome H
∘ les sulfonylimidures d'immidazolium Ieᵢₘ.

Une 6^{ème} famille de sulfonylimidures comprend les composés dans lesquels le cation M est divalent, à savoir les composés (If) (Ar-Z-L-SO₂N-SO₂R')⁻₂M²⁺. On peut citer en particulier les sulfonylimidures de calcium If_{ca} et de magnésium If_{mg}.

Une 7^{ème} famille de sulfonylimidures comprend les composés (Ig) qui répondent à la formule (I) dans laquelle Ar représente un groupe phényle portant éventuellement au moins un substituant, en particulier un groupe phényle sans substituant, ou un groupe portant un substituant choisi parmi :
- les halogènes (par exemple Br ou F) ;
- les trialkylsilyles (par exemple le triméthylsilyle) ;
- le groupement hydroxyle et les groupes éther (par exemple le groupe benzyloxy PhCH₂O-, le groupe triphényloxy Ph₃CO-, le groupe méthyloxy CH₃O-), ester carboxylique (par exemple le groupe benzoyloxy PhC(O)O- et le groupe acétyloxy CH₃C(O)O-) ; les atomes d'halogène, Cl-CH₂-, et les groupes Q¹-O-CH₂- dans lesquels Q¹ est H, un groupe alkyle ou un groupe acyle ;
- les groupements possédant une insaturation éthylénique [provenant par exemple d'un motif vinyle (CH=CH₂-), d'un motif allyle (CH₂=CH-CH₂-) ou d'un motif acryloyloxy (CH₂=CH-C(O)-O-)], éventuellement substitué ;-les groupements amino -N(Q³) (Q⁴) - dans lesquels Q³ et Q⁴ représentent chacun indépendamment de l'autre H, un groupe alkyle, un groupe aryle, un groupe arylalkyle ou un groupe acyle, [par exemple CH₃C(O)NH- ou PhCH₂NH-)]. Une 8^{ème} famille de sulfonylimidures comprend les composés (Ih) qui répondent à la formule (I) dans laquelle Ar représente un groupe aromatique hétérocyclique, en particulier un groupe pyridine.

Les N-benzyl-sulfimides et les N-allyl-sulfimides obtenus en tant que produits intermédiaires répondent à la formule RSO₂N(R")SO₂R' (II) dans laquelle R a la signification la plus générale donnée précédemment, R" est un groupe benzyle ou allyle, et R' est un groupe perfluoroalkyle linéaire ou ramifié, de préférence un groupe CF₃ ou C₂F₅.

Un fluorure de sulfonyle (III) est obtenu par un procédé comprenant une étape de préparation d'un sulfinate [Ar-Z-(CF₂)ₙ-CFR_{f}-SO₂]ₘM (IV), et une étape de transformation du sulfinate en fluorure de sulfonyle (III), Ar, Z et n ayant la signification précédente, M étant un cation de métal alcalin ou d'ammonium.

Les modalités de mise en oeuvre de l'étape de préparation du sulfinate dépendent du sulfinate visé, et notamment de la nature de Z du groupement Ar-Z qu'il contient.

Un sulfinate Ar-S-(CF₂)ₙ-CFR_{f}-SO₂ M (désigné ci-après par IVₛ) dans lequel Ar, -(CF₂)ₙ-CFR_{f}- et M ont la signification donnée ci-dessus peut être obtenu par un procédé comprenant :
- une première étape consistant à réduire un halogénure ArS-(CF₂)ₙ-CFR_{f}-X dans lequel X représente Br ou Cl, par un excès de magnésium en présence de chlorure de triméthylsilyle (TMSCl) pour obtenir un composé Ar-S-(CF₂)ₙ-CFR_{f}-Si-(CH₃)₃ ;
- une deuxième étape au cours de laquelle on fait réagir le composé Ar-S-(CF₂)ₙ-CFR_{f}-Si-(CH₃)₃ avec un fluorure du cation M en présence de SO₂ pour obtenir le sulfinate.

La 1^{ère} étape est effectuée de préférence dans le THF, et la 2^{ème} étape de préférence dans MeCN.

Un composé du type Ar-S-(CF₂)ₙ-CFR_{f}-Br peut être obtenu par la réaction du thiol Ar-SH avec un dibromure Br-(CF₂)ₙ-CFR_{f}-Br en utilisant le dibromure en excès.

Un sulfinate Ar-SO-(CF₂)ₙ-CFR_{f}-SO₂M (désigné ci-après par IV_{SO}) ou un sulfinate Ar-SO₂-(CF₂)ₙ-CFR_{f}-SO₂M (désigné ci-après par IV_{SO2}) peuvent être obtenus par un procédé comprenant
- une 1^{ère} étape consistant à réduire un halogénure Ar-S-(CF₂)ₙ-CF_{R}f-X dans lequel X représente F, Br ou Cl, par un excès de magnésium en présence de chlorure de triméthylsilyle (TMSCl) pour obtenir un composé Ar-S-(CF₂)ₙ-CFR_{f}-Si-(CH₃)₃ ;
- une 2^{ème} étape au cours de laquelle on oxyde le composé Ar-S-(CF₂)ₙ-CFR_{f}-Si-(CH₃)₃ à l'aide d'acide métachloroper-benzoïque (m-CPBA) en excès, la quantité d'acide étant choisie en fonction du degré d'oxydation [(IV) ou (VI)] que l'on souhaite atteindre pour l'atome de S, l'oxydation étant effectuée avantageusement dans le dichlorométhane anhydre ;
- une 3^{ème} étape au cours de laquelle on condense avec le dioxyde de soufre en présence de MF, le silane obtenu à la fin de la 2^{ème} étape.

Les modalités de mise en oeuvre de l'étape d'obtention d'un fluorure de sulfonyle à partir d'un sulfinate dépendent du fluorure de sulfonyle visé, notamment de la nature de Z du groupe Ar-Z qu'il contient dans la formule générale III.

Un fluorure de sulfonyle III peut être obtenu par réaction du sulfinate IV correspondant avec un agent de fluoration choisi parmi le fluor, le difluorure de xénon, le fluorosulfate de potassium, le N-fluorobenzènesulfonimide l'heptadiborate de N-fluoropyridinium, le trifluorométhanesulfonate de N-fluoropyridinium, le bis-tétrafluoroborate de N,N'-difluoro-2,2'-bipyridinium, et le le bis(tétrafluoroborate) de 1-chlorométhyl-4-fluoro-1,4-diazoniabicyclo-[2,2,2] octane (F-TEDA). La réaction peut être mise en oeuvre à l'aide de sulfinate en solution dans le solvant dans lequel il a été obtenu, si ledit solvant est l'acétonitrile.

Les agents de fluoration utilisés pour la fluoration du sulfinate sont des produits disponibles dans le commerce. Par exemple, le bis(tétrafluoroborate) de 1-chlorométhyl-4-fluoro-1,4-diazoniabicyclo[2,2,2]octane (F-TEDA) répondant à la formule 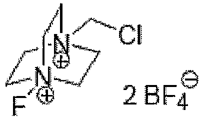 est commercialisé sous la dénomination Selectfluor® par la société Air Products. Le fluor F₂, le difluorure de xénon XeF₂, le fluorosulfate de potassium KSO₄F, le N-fluorobenzènesulfonimide (PhSO₂)₂NF, l'heptafluorodiborate de N-fluoropyridinium 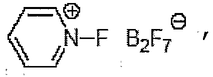 le trifluorométhanesulfonate de N-fluoropyridinium 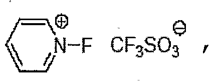 et le bis-tétrafluoroborate de N,N'-difluoro-2,2'-bipyridinium 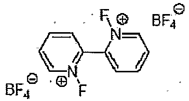 sont commercialisés notamment par la société Acros et la société Aldrich.

Ledit procédé peut être mis en oeuvre avantageusement pour obtenir les fluorures de sulfonyle suivants :
- Ar-S-(CF₂)ₙ-CFR_{f}-SO₂F (III_{S})
- Ar-SO-(CF₂)ₙ-CFR_{f}-SO_{2F} (III_{SO})
- Ar-SO₂(CF₂)ₙ-CFR_{f}-SO₂F (III_{SO2}).

Les fluorures de sulfonyle III_{SO} et III_{SO2} peuvent être obtenus par oxydation du fluorure Iₛ correspondant. L'oxydation est effectuée avantageusement à l'aide d'acide méta-chloroperbenzoïque (m-CPBA) en excès, la quantité d'acide étant choisie en fonction du degré d'oxydation (IV) ou (VI)] que l'on souhaite atteindre pour l'atome de S.

De manière générale, lorsqu'un halogénure de départ ArS-(CF₂)ₙ-CFR^{f}-X n'est pas disponible pour un groupement Ar particulier, le groupement Ar' d'un halogénure existant peut être modifié sur le composé Ar'-S-(CF2)ₙ-CFR^{f}-Si (CH₃)₃ (III), sur le sulfinate [Ar'-S-(CF2)ₙ-CFR^{f}-SO₂]ₘM (II) ou sur le fluorure de sulfonyle Ar'-S-(CF₂)ₙ-CFR^{f}-SO₂F (I)

Le tableau suivant donne quelques exemples de réactions utiles, dont la mise en oeuvre et la généralisation sont à la portée de l'homme de métier.

| Ar' | Ar | Réaction |
|---|---|---|
| Br-Ph | (CH₃)₃Si-Ph | Réaction d'un composé IV (Ar' est Br-Ph et X est Br) avec Mg et TMSCI |
| CH₃OH-Ph | HO-Ph | Réaction d'un composé I (Ar' est CH₃OH-Ph) avec BBr₃, puis NH₄OH |
| HO-Ph | CH₂=CH-C(O)-O-Ph | Réaction d'un composé I (Ar' est HO-Ph) avec le chlorure d'acryloyle et la diisopropyléthylamine |
| (CH₃)₃Si-Ph | I-Ph | Réaction d'un composé III (Ar' est (CH₃)₃Si-Ph) avec ICl |
| I-Ph | CH₂=CH-Ph | Réaction d'un composé III (Ar' est (I-Ph) avec le bromure de vinylmagnésium en présence de PdCl₂(PPh₃)₂ |
| F-Ph | RO-Ph | Réaction d'un composé I (Ar' est F-Ph et Z = SO₂) avec un alcoolate. |
| F-Ph | Amino-Ph | Réaction d'un composé I (Ar est F-Ph et Z = SO₂ avec un amidure ou un composé libérant un amidure, par exemple le diméthylformamide |

Les sulfonylimidures (I) obtenus selon le procédé de la présente invention peuvent entrer dans la composition d'électrolytes utilisables dans divers dispositifs électrochimiques.

Les sulfonylimidures I_{Li} sont particulièrement utiles comme sels pour l'électrolyte de dispositifs électrochimiques tels que les piles au lithium non rechargeable, les accumulateurs lithium polymère, les accumulateurs lithium-ion, les supercondensateurs organiques et les dispositifs électrochromes.

Dans les piles au lithium, les accumulateurs lithium-ion et les supercondensateurs organiques, l'électrolyte peut être un électrolyte liquide contenant le sel I_{Li} en solution dans un solvant aprotique polaire. Le solvant liquide aprotique, utilisé seul ou en mélange, est choisi par exemple parmi les éthers linéaires et les éthers cycliques, les esters, les nitriles, les dérivés nitrés, les amides, les sulfones, les sulfolanes, les alkylsulfamides, les carbonates linéaires et cycliques. Les solvants particulièrement préférés sont le diéthyléther, le diméthoxyéthane, le glyme, le tétrahydrofurane, le dioxane, le diméthyltétrahydro-furane, le formiate de méthyle ou d'éthyle, le carbonate de propylène ou d'éthylène, les carbonates d'alkyles (notamment le carbonate de diméthyle, le carbonate de diéthyle et le carbonate de méthylpropyle), les butyrolactones, l'acéto-nitrile, le benzonitrile, le nitrométhane, le nitrobenzène, la diméthylformamide, la diéthylformamide, la N-méthylpyrro-lidone, la diméthylsulfone, la tétraméthylène sulfone et les tétraalkylsulfonamides ayant de 5 à 10 atomes de carbone.

L'électrolyte liquide peut être utilisé avec un séparateur microporeux ou macroporeux dont il remplit la porosité. Le séparateur peut être de type PVdF, polysulfone, polyimide, Celgard® ou des tissus Gore®. L'électrolyte liquide peut être utilisé pour gonfler un polymère non poreux, formant ainsi un électrolyte polymère plastifié. L'électrolyte polymère plastifié contient au moins 80% en poids d'électrolyte liquide et au plus 20% en poids de polymère. Pour les électrolytes polymères plastifiés, on utilise de préférence un polymère tridimensionnel à base, par exemple, de poly(oxyéthylène), de PVDF-HFP, de polyacrylonitrile, de polyméthacrylate, de polyacrylate, de polyméthacrylonitrile ou de réseaux semi interpénétré des dits polymères.

Les sulfonylimidures de cations organiques, et plus particulièrement les sulfonylimidures d'ammonium Iₐₘₘ obtenus selon le procédé de la présente invention sont particulièrement utiles comme liquides ioniques dans divers dispositifs électrochimiques, notamment les piles au lithium non rechargeables, les accumulateurs lithium-ion, les supercondensateurs organiques et les dispositifs électrochromes.

Les sulfonylimidures Iₐₘₘ constituent des liquides ioniques qui peuvent avantageusement remplacer un solvant ou mélange de solvants liquides organiques.

Les composés Iₐₘₘ peuvent être utilisés à l'état pur comme liquides ioniques dans un dispositif électrochimique comme un supercondensateur organique, en particulier avec un séparateur microporeux ou macroporeux mentionnés précédemment.

Les composés Iₐₘₘ peuvent être utilisés en solution dans un solvant liquide aprotique tel que défini ci-dessus, pour former un électrolyte liquide utilisable en particulier dans un supercondensateur organique, en particulier avec un séparateur microporeux ou macroporeux de type PVdF, polysulfone, polyimide, Celgard® ou des tissus Gore®. Les composés Iₐₘₘ peuvent être dissous dans une membrane polymère dense éventuellement gonflée par un solvant organique. Ledit polymère peut être un polymère fonctionnel capable d'absorber de fortes quantités de composés Iₐₘ. Pour les électrolytes polymères dense gonflés, on utilise de préférence un polymère tridimensionnel à base, par exemple, de poly(oxyéthylène), de PVDF-HFP, de polyacrylonitrile, de polyméthacrylate, de polyacrylate, de polyméthacrylonitrile, ou de réseaux semi interpénétré desdits polymères ou un polymère fonctionnalisé à haut Tg tel que les polyéthersulfones fonctionnalisées. Lorsque l'on plastifie un polymère par un électrolyte liquide à base de liquides ioniques de type Iₐₘ, l'électrolyte polymère plastifié contient au moins 60% en poids d'électrolyte liquide et au plus 40% en poids de polymère.

Les liquides ioniques Iₐₘ peuvent être utilisés en mélange avec un sel de lithium (par exemple un composé I_{Li}) dans les piles au lithium, les accumulateurs lithium-ion ou les supercondensateurs organiques. On peut le cas échéant, pour diminuer la viscosité, additionner un solvant aprotique polaire tel que défini ci-dessus. Les solutions de sel dans un liquide ionique sont utilisées avantageusement avec un séparateur microporeux ou macroporeux, tel que défini ci-dessus.

Pour les électrolytes polymères plastifiés, on utilise de préférence un polymère tridimensionnel à base, par exemple, de poly(oxyéthylène), de PVDf-HFP, de polyacrylonitrile, de polyméthacrylate, de polyacrylate, de polyméthacrylonitrile ou de réseaux semi interpénétré des dites polymères.

Un composé obtenu selon procédé de la présente invention peut être utilisé sous forme d'électrolyte polymère comprenant le sulfonylimidure I_{Li} en solution dans un polymère solvatant plastifié ou non par un solvant liquide polaire ou un liquide ionique Iₐₘ.

Le solvant polymère peut être choisi parmi les polymères solvatants, réticulés ou non, portant ou non des groupes ioniques greffés. Un polymère solvatant est un polymère qui comporte des unités solvatantes contenant au moins un hété-roatome choisi parmi le soufre, l'oxygène, l'azote. A titre d'exemple de polymères solvatants, on peut citer les polyéthers de structure linéaire, peigne ou à blocs, formant ou non un réseau, à base de poly(oxyéthylène)ou les copolymères contenant le motif oxyde d'éthylène ou oxyde de propylène ou allylglycidyléther, les polyphosphazènes et poly-siloxanes ayant des chaînes latérales d'oligo(oxyéthylène), les réseaux réticulés à base de polyéthylène glycol réticulé par des isocyanates ou les réseaux obtenus par polycondensation et portant des groupements qui permettent l'incorporation de groupements réticulables. On peut également citer les copolymères à blocs dans lesquels certains blocs portent des fonctions qui ont des propriétés rédox. Bien entendu, la liste ci-dessus n'est pas limitative, et tous les polymères présentant des propriétés solvatantes peuvent être utilisés.

Lorsque le solvant de l'électrolyte est un polymère solvatant plastifié, il contient au moins 80% de polymère et au plus 20% de liquide polaire ou de liquide ionique Iₐₘ.

Une batterie lithium-polymère comprend une électrode négative et une électrode positive séparées par un électrolyte solide polymère. L'électrode positive d'une batterie lithium-polymère est constituée par une matière active d'électrode positive, éventuellement un liant et un matériau conférant une conduction électronique. Dans une batterie rechargeable au lithium, l'anode est constituée par un film de lithium métallique, d'un alliage de lithium ou d'un composé intermétallique de lithium. Dans une batterie de type lithium-ion, l'électrode négative est constituée par un matériau capable d'insérer de manière réversible des ions lithium tel que le graphite par exemple. Le sulfonylimidure obtenu selon le procédé de l'invention utilisé pour l'électrolyte peut être un sulfonylimidure Iₗᵢ ou un sulfonylimidure Iₗᵢ mélangé à un sulfonylimidure Iₐₘₘ. A ces sulfonylimidures peuvent être ajoutés des sels de lithium de type A-,Li⁺. La propriété recherchée est alors de combiner le fort nombre de transport cationique des sulfonylimidures Iₗᵢ aux fortes conductivités d'autres sels Parmi les anions on peut citer de manière non exhaustive ClO₄⁻, BF₄⁻, PF₆⁻, AsF6⁻, (CF₃SO₂)₂N⁻, (CF₃SO₂)₂CH (CF₃SO₂)₃C⁻·

L'électrolyte polymère peut être renforcé par addition de charges de renforts comme les microfibrilles de cellulose.

Dans les batteries Ca ou les batteries Mg, on utilise pour l'électrolyte, respectivement un sulfonylimidure I_{ca} ou I_{mg} tels que définis ci-dessus. L'électrode négative est constituée par le métal Ca ou Mg et selon Novak et al. JPS 1995, on peut utiliser des vanadates de type Mg(V₃O₈).

Dans une batterie protonique, l'électrolyte contient avantageusement un sulfonylimidure Iₐₕ.

Un supercondensateur est constitué par une cellule électrochimique comprenant deux électrodes séparées par un électrolyte, dans laquelle le matériau constituant les électrodes présente une très grande aire spécifique (par exemple de 100 à 1500 m²/g). Lorsque l'électrolyte d'un supercondensateur comprend un sulfonylimidure obtenu selon le procédé de la présente invention, ledit sulfonylimidure est choisi de préférence parmi les composés Iₐₘₘ ou I_{Li} pour les supercondensateurs organiques ou Iₐₕ pour les supercondensateurs aqueux.

Une pile à combustible est un dispositif électrochimique constitué par une anode et une cathode séparées par un électrolyte. Les piles à combustible dites PEMFC (Proton Exchange Membrane Fuel Cell) sont des piles à combustible à membrane qui utilisent de l'hydrogène stocké en bouteille ou de l'hydrogène issu d'un reformage de méthanol. Les piles à combustible à méthanol direct dites DMFC (Direct Méthanol Fuel Cell) utilisent le méthanol dans la solution d'électrolyte. L'utilisation des PEMFC est limitée à des températures inférieures ou égales à 85°C. A plus haute température, l'eau s'évapore et la conductivité des ionomères diminue. Il est donc intéressant de trouver des conducteurs protoniques anhydres capables de conduire au-delà de 130°C. Les composés Iₐₕ sont suffisamment conducteurs à l'état pur. Ils peuvent être utilisés en mélange avec un excès d'acide ou d'amine ayant servi à les préparer ou (et) en présence de Iₐₘ. Si leurs viscosités sont trop fortes ils peuvent être mélangés à des solvants organiques polaires comme le sulfolane ou la tétraéthylsulfonamide. Pour assurer la tenue mécanique, l'électrolyte peut être utilisé avec un séparateur micro- ou macroporeux tel que défini précédemment, dont il imprègne la porosité. L'électrolyte peut être une membrane d'un polymère non poreux gonflé par Iₐₕ. Le choix d'un polymère fonctionnel est essentiel pour optimiser la prise de Iₐₕ dans la membrane. Le polymère fonctionnel peut être un polymère non ionique ou un ionomère tel que le Nafion ou les polyéthersulfones sulfonées.

Un vitrage électrochrome est une cellule électrochimique comprenant deux électrodes séparées par un électrolyte. L'une des électrodes est une électrode transparente, l'autre électrode peut être constituée par exemple par un oxyde de tungstène WO₃ déposé sur un film d'oxyde d'étain ITO. Sous l'action d'un courant, la couleur de l'électrode WO₃ est modulée et passe de l'incolore au bleu foncé par insertion de protons. Pour cette application particulière on utilise de préférence un sulfonylimidure Iₐₕ.

La présente invention est illustrée par les exemples concrets décrits ci-après, auxquels elle n'est cependant pas limitée.

### Exemple 1

### [(2-Bromo-1,1,2,2-Tétrafluoroéthyl)sulfanyl]benzène

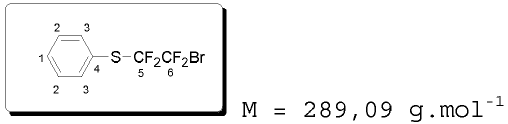

Dans un ballon tricol placé sous azote, surmonté d'un réfrigérant à carboglace et d'une ampoule de coulée, le thiophénol (10,2 mL, 100 mmol) est additionné goutte à goutte (30 min) à 0°C sur une suspension de NaH (6 g, 150 mmol) dans du DMF anhydre (100 mL). Le mélange est ensuite agité à cette température pendant 20 min puis refroidi à -50°C. Le 1,2-dibromo-1,1,2,2-tétrafluoroéthane (15 mL, 125 mmol) est alors ajouté goutte à goutte à -50°C en 10 min. Le mélange est ensuite agité pendant 2 h à cette température puis 1 h à température ambiante. De l'eau (150 mL) est ajoutée au mélange réactionnel puis le produit est extrait par l'éther éthylique (3 x 100 mL). Les phases organiques sont lavées par de l'eau (3 x 100 mL) et séchées sur MgSO₄. Après évaporation du solvant, le résidu est purifié par distillation sous pression réduite (99°C/40mmHg). Le [(2-Bromo-1,1,2,2-Tétrafluoroéthyl)-sulfanyl]benzène est alors obtenu sous forme d'un liquide incolore (26,07 g, 90%).
- **CCM :**: *R^{f}* 0,8 (pentane)
- **RMN** ¹⁹**F**: (282 MHz, CDCl₃) : δ -62,61 (t, 2F, CF₂Br, ³*J*_{F-F} = 8,0 Hz), -85,57 (t, 2F, SCF₂, ³*J*_{F-F} = 8,0 Hz).
- **RMN** ¹**H**: (300 MHz, CDCl₃) : δ7.38-7.44 (m, 2H, H₂), 7,47-7,52 (m, 1H, H₁), 7, 64-7,66 (d, 2H, H₃,³*J*_{H2}-_{H3} = 7,1 Hz).
- **RMN** ¹³**C**: (75 MHz, CDCl₃) : δ 116,93 (tt, 1C, CF₂, ¹*J*_{F-C}= 312,9 Hz, ²*J*_{F-C} = 40,6 Hz), 122,78 (tt, 1C, CF₂, ¹*J*_{F-C} = 290,7 Hz, ²*J*_{F-C} = 33,8 Hz), 123,6 (t, 1C, C _{Ar 4}, ³*J*_{F-C} = 2,7 Hz), 129,53 (s, 2C, C_{Ar 2}), 131,09 (s, 1C, C_{Ar 1}), 137,42 (s, 2C, C_{Ar 3}).
Les carbones CAr 1, CAr 2 et CAr 3 sont déterminés sur les spectres de RMN 2D par analogie avec des produits de la même famille.

### Exemple 2

### [(2-phénylsulfanyl)-1,1,2,2-tétrafluoroéthyl]triméthylsilane

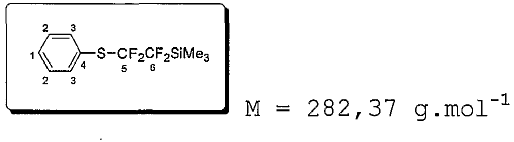

Sous atmosphère inerte, PhSCF₂CF₂Br (3,7 g, 12,8 mmol) préparé selon le mode opératoire de l'exemple 1, est ajouté goutte à goutte à une suspension de tournures de magnésium (615 mg, 25,6 mmol), de chlorure de triméthylsilyle (6,5 mL, 61 mmol) et de THF anhydre (25 mL), refroidie à -20°C. Le mélange est agité à -20°C pendant 1 h puis à température ambiante pendant 5 h puis concentré. Le solide résultant est lavé avec du pentane et le filtrat est évaporé pour donner le [(2-phénylsulfanyl)-1,1,2,2-tétrafluoroéthyl]triméthyl- silane sous forme d'un liquide jaune (3,3 g, 90%).
- **CCM :**: *R^{f}* 0,7 (pentane)
- **RMN** ¹⁹**F**: (282 MHz, CDCl₃) : δ -82,88 (t, 2F, SCF₂, ³*J*_{F-F} = 4,6 Hz), -122,56 (t, 2F, CF₂Si, ³*J*_{F-F} = 4, 6 Hz).
- **RMN ¹H**: (300 MHz, CDCl₃) : δ 0,27 (s, 9H, Si(CH₃)₃), 7,36-7,48 (m, 3H, H₂ et H₁), 7,64-7,66 (m, 2H, H₃).
- **RMN** ¹³**C**: (75 MHz, CDCl₃) : δ -3,98 (m, 3C, Si(CH₃)₃), 123,01 (tt, 1C, CF₂, ¹*J*_{F*-*C} = 273,2 Hz, ³*J*_{F-C} = 45,3 Hz), 124,63 (m, 1C, C _{Ar 4}), 127,54 (tt, 1C, CF₂, ¹*J*_{F-C} = 281,8 Hz, ³*J*_{F-C} = 32,5 Hz), 129,21 (s, 2C, C_{Ar 2}), 130,29 (s, 1C, C_{Ar 1}), 137,31 (s, 2C, C_{Ar 3}).
(Carbone non attribué précisément, mais par analogie avec des produits de la même famille.)

### Exemple 3

### Fluorure de 2-(Phénylsulfanyl)-1,1,2,2-tétrafluoroéthanesulfonyle

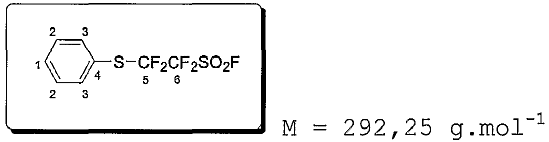

Une solution de dioxyde de soufre est préparée par bullage de dioxyde de soufre (1,02 g, 16 mmol) dans une solution d'acétonitrile anhydre (20 mL) à température ambiante. Cette solution est ajoutée à PhSCF₂CF₂SiMe₃ (8 mmol, préparé selon le mode opératoire de l'exemple 2) et agitée à température ambiante sous atmosphère inerte. Le CsF anhydre (1,4 g, 9 mmol) est ensuite ajouté sur le mélange réactionnel qui est agité à température ambiante pendant une nuit. La réaction est suivie par CCM et par RMN ¹⁹**F** (CDCl₃) jusqu'à disparition de PhSCF₂CF₂SiMe₃. Le F-TEDA (2,9 g, 8,2 mmol) est ajouté sur le mélange qui est agité 1 h à température ambiante. Le mélange est concentré et le résidu solide lavé par de l'éther éthylique (10 x 50 mL). Le filtrat est évaporé et le produit purifié par distillation au four à boules. Le Fluorure de 2-(Phénylsulfanyl)-1,1,2,2- tétrafluoroéthanesulfonyle est obtenu sous forme de liquide incolore (1,86 g, 79%).
- **CCM :**: *R^{f}* 0,7 (pentane)
- **RMN** ¹⁹**F**: (282 MHz, CDCl₃) : δ 45,93-46,02 (m, 1F, SO₂F), -86, 52-86, 59 (m, 2F, SCF₂), -105,567 (m, 2F, CF₂SO₂F).
- **RMN** ¹**H**: (300 MHz, CDCl₃) : δ7.44(dd, 2H, H₂, ³*J*_{H2-H3} = ³*J*_{H2-H1} = 7.4 Hz), 7,54 (t, 1H, H₁, ³*J*_{H1-H2} = 7,4 Hz), 7.67 (d, 2H, H₃, ³*J*_{H2-H3} = 7,4 Hz).
- **RMN** ¹³**C**: (75 MHz, CDCl₃) : δ 116,15 (ttd, 1C, C₆, ¹*J*_{F-C} = 300,6 Hz, ²*J*_{F-C} = 40,8 Hz, ²*J*_{F-C} = 32,6 Hz), 121.78 (ttd, 1C, C₅, ¹*J*_{F-C} = 290, 9 Hz, ²*J*_{F-C} = 31,7 Hz, ³*J*_{F-C} = 1,2 Hz), 122,01 (t, 1C, C _{Ar 4}, ³*J*_{F-C} = 3,6 Hz), 129,80 (s, 2C, C_{Ar 2}), 131,68 (s, 1C, C_{Ar 1}), 137,53 (S, 2C, C_{Ar 3}).
CAr 1, CAr 2 et CAr 3 sont déterminés par RMN 2D.

### Exemple 4

### Fluorure de 2-(phénylsulfonyl)-1,1,2,2-tétrafluoro-éthanesulfonyle

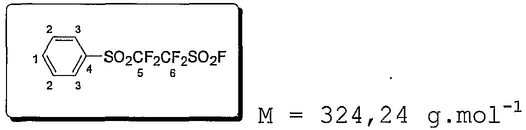

A une solution de PhSCF₂CF₂SO₂F (1,32 g, 4,5 mmol, obtenu selon le mode opératoire de l'exemple 3) dans du dichlorométhane anhydre (45 mL) est ajouté de l'acide métachloroperbenzoique (6,25 g, 36 mmol). Le milieu réactionnel est agité à température ambiante pendant 2 jours (suivi par RMN ¹⁹F) puis filtré sur gel de silice et lavé par du CH₂Cl₂. Après évaporation du solvant, le résidu est purifié par chromatographie sur gel de silice (Pentane/ CH₂Cl₂, 4/1). Le Fluorure de 2-(phénylsulfonyl)-1,1,2,2-tétrafluoro-éthanesulfonyle est obtenu sous forme de liquide incolore (1,15 g, 79%).
- CCM :: *R^{f}* 0,7 (Pentane/CH₂Cl₂, 1/1)
- **RMN** ¹⁹**F**: (282 MHz, CDCl₃) : δ 46, 20-46, 30 (m, 1F, SO₂F), -106,27 ; -106,37 (m, 2F), -110,72 ;-110,83 (m, 2F).
- **RMN** ¹**H**: (300 MHz, CDCl₃) : δ 7.71 (dd, 2H, H₂, ³*J*_{H2-H3} = ³*J*_{H2-H1} = 7,5 Hz), 7,89 (t, 1H, H₁, ³*J*_{H1-H2} = 7,5 Hz), 8,06 (d, 2H, H₃, ³*J*_{H2-H3} = 7,5 Hz).
- **RMN** ¹³**C**: (75 MHz, CDCl₃) : δ 113,55 (tt, 1C, C₅, ¹*J*_{F-C} 302,0 Hz, ³*J*_{F-C} = 35,0 Hz), 115,41 (ttd, 1C, C₆, ¹*J*_{F-C} = 302,0 Hz, ³*J*_{F-C} = 35,0 Hz, ³*J*_{F-C} = 35,0 Hz), 130,14 (s, 2C, C_{Ar 2}), 131,35 (s, 2C, C_{Ar 3}), 131,77 (s, 1C, C _{Ar 4})_{,} 137,20 (s, 1C, C_{Ar 1}) .

### Exemple 5

### [(Bromodifluorométhyl)sulfanyl]benzène

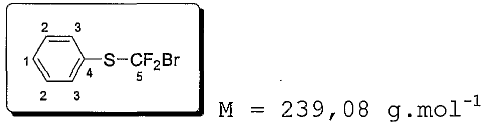

Dans un ballon tricol placé sous atmosphère d'azote, surmonté d'un réfrigérant à carboglace et d'une ampoule de coulée, le thiophénol (10,2 mL, 100 mmol) est additionné goutte à goutte, en 40 min à 0°C, sur une suspension de NaH (6 g, 150 mmol) dans du DMF anhydre (100 mL). Le mélange est ensuite agité à 0 °C pendant 30 min puis refroidi à -50°C. Le dibromodifluorométhane (27 mL, 300 mmol) est alors ajouté à -50°C. Le mélange est ensuite agité pendant 3 h à cette température puis 30 min à température ambiante. De l'eau (100 mL) est ajoutée au mélange réactionnel puis le produit est extrait par l'éther éthylique (3 x 100 mL). Les phases organiques sont lavées par de l'eau (3 x 100 mL) et séchées sur MgSO₄. Après évaporation du solvant, le résidu est distillé sous pression réduite (97°C/34mmHg) pour fournir le [(Bromodifluorométhyl)sulfanyl]benzène sous forme d'un liquide incolore (15,3 g, 60%)
- **CCM :**: *R^{f}* 0,7 (pentane)
- **RMN** ¹⁹**F**: (282 MHz, CDCl₃) : δ -22,53 (s, 2F)
- **RMN** ¹**H**: (300 MHz, CDCl₃) : δ 7,43 (m, 2H, H₂), 7,52 (m, 1H, H₁), 7,66 (d, 2H, H₃, ³*J*_{H2-H3} = 7,4 Hz).
- **RMN** ¹³**C**: (75 MHz, CDCl₃) : δ 119,41 (t, 1C, CF₂, ¹*J*_{F-C} = 336,0 Hz), 127, 30 (t, 1C, C_{Ar 4}, ³*J*_{F-C} = 1,1 Hz), 129,58 (s, 2C, C_{Ar 2}), 131,18 (s, 1C, C_{Ar 1}), 136,52 (s, 2C, CAr ₃).

### Exemple 6

### difluoro(phénylsulfanyl)méthyl]triméthylsilane

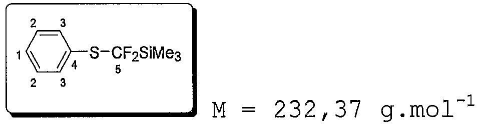

Sous atmosphère inerte, PhSCF₂Br (4,8 g, 20 mmol, obtenu selon le mode opératoire de l'exemple 5) est ajouté goutte à goutte à une suspension de tournures de magnésium (960 mg, 40 mmol), de chlorure de triméthylsilyle (10,2 mL, 80 mmol) et de THF anhydre (50 mL) refroidie à 0°C. Le mélange est encore agité à 0°C pendant 1 h puis à température ambiante pendant 1 h. Le mélange est ensuite concentré et le solide résultant est lavé à l'éther de pétrole. La phase organique est évaporée sous vide pour fournir le difluoro(phényl-sulfanyl)méthyl]triméthylsilane sous forme d'un liquide jaune (4,3 g, 92%).
- **CCM :**: *R^{f}* 0,5 (pentane)
- **RMN** ¹⁹**F**: (282 MHz, CDCl₃) : δ -88,01 (s, 2F)
- **RMN** ¹**H**: (300 MHz, CDCl₃) : δ 0,25 (s, 9H, Si (CH₃)₃), 7,35-7-40 (m, 3H, H₂ et H₁), 7,58-7,61 (m, 2H, H₃).
- **RMN** ¹³**C**: (75 MHz, CDCl₃) : δ -4,08 (t, 3C, Si(CH₃)₃, ³*J*_{F-C} = 1,3 Hz), 126,44 (t, 1C, C _{Ar 4}, ³*J*_{F-C} = 4,1 Hz), 128,93 (s, 2C, C_{Ar 2}), 129, 40 (s, 1C, C_{Ar 1}), 134,10 (t, 1C, CF₂, ¹*J*_{F-C} = 300,2 Hz), 136,30 (t, 2C, C_{Ar 3}, ⁴*J*_{F-C} = 1,1 Hz).

### Exemple 7

### Fluorure de difluoro(phénylsulfanyl)méthanesulfonyle

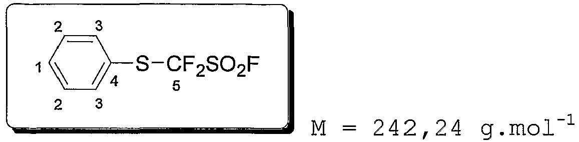

Une solution de dioxyde de soufre est préparée par bullage de dioxyde de soufre (3,9 g, 61 mmol) dans une solution d'acétonitrile anhydre (20 mL) à température ambiante. Cette solution est ajoutée sur PhSCF₂SiMe₃ (4,3 g, 18,4 mmol, obtenu selon le mode opératoire de l'exemple 6) et agitée à température ambiante sous atmosphère inerte. Le CsF anhydre (2,8 g, 18,5 mmol) est ensuite ajouté sur le mélange réactionnel qui est agité à température ambiante pendant une nuit. La réaction est suivie par CCM et par RMN ¹⁹F (CDCl₃) jusqu'à disparition de PhSCF₂SiMe₃. Le F-TEDA (7,15 g, 20 mmol) est ajouté sur le mélange à -20°C qui est ensuite agité 1 h à température ambiante. Le mélange est concentré et le résidu solide lavé par de l'éther éthylique (10 x 50 mL). Le filtrat est évaporé et le produit purifié par distillation au four à boules. Le fluorure de difluoro-(phénylsulfanyl)méthanesulfonyle est obtenu sous forme de liquide incolore (2,5 g, 57%).
- **CCM :**: *R^{f}* 0,6 (pentane)
- **RMN** ¹⁹**F**: (282 MHz, CDCl₃) : δ 35,06 (t, 1F, SO₂F, ³*J*_{F-F} = 4,6 Hz), -76,08 (d, 2F, -SCF₂-, ³*J*_{F-F} = 4,6 Hz).
- **RMN** ¹**H**: (300 MHz, CDCl₃) : δ 7,46 (dd, 2H, H₂, ³*J*_{H2-H1} = ³*J*_{H2-H3} = 7,5 Hz), 7,56 (t, 1H, H₁, ³*J*_{H1-H2} = 7,5 Hz), 7,71 (d, 2H, H₃, ³*J*_{H3-H2} = 7,5 Hz).
- **RMN** ¹³**C**: (75 MHz, CDCl₃) : δ 121,83 (t, 1C, C _{Ar 4}, ³*J*_{F-C} = 3,0 Hz), 126,56 (td, 1C, CF₂, ¹*J*_{F-C} = 323,3 Hz, ²*J*_{F-C} = 32,9 Hz), 129,95 (s, 2C, C_{Ar 2}), 132,13 (s, 1C, C_{Ar 1}), 137.52 (t, 2C, C_{Ar 3}, ⁴*J*_{F-C} = 1.1 Hz).

### Exemple 8

### N-Benzyl-2-(phénylsulfanyl)-1,1,2,2-tétrafluoro-éthanesulfonamide

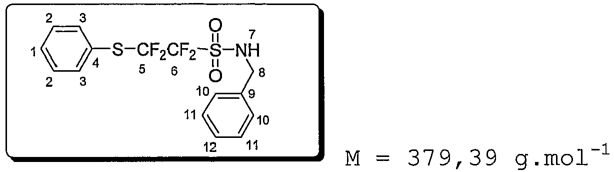

A une solution de 1,2-dichloroéthane (20 mL), contenant le PhSCF₂CF₂SO₂F (2,09 g, 7,2 mmol) obtenu selon le procédé de l'exemple 3, on ajoute de la benzylamine fraichement distillée (4 mL, 37 mmol). Le mélange est agité et chauffé à 50°C pendant 20 h jusqu'à disparition de PhSCF₂CF₂SO₂F (suivi par CCM et RMN ¹⁹F/CDCl₃). Après retour à température ambiante, une solution aqueuse de HCl (10%) est ajoutée au milieu réactionnel. Les phases aqueuses et organiques sont séparées et la phase aqueuse est extraite par du dichlorométhane. Les phases organiques sont regroupées et séchées sur MgSO₄. Après filtration et évaporation des solvants, le résidu est purifié par chromatographie sur gel de silice (Pentane à Pentane/AcOEt 4/1). Le sulfonamide est obtenu sous forme de solide blanc (2,1 g, 77%).
- **CCM** :: *R^{f}* 0,8 (pentane/AcOEt 4/1)
- **RMN** ¹⁹**F**: (282 MHz, CDCl₃) : δ -85,29 (t, 2F, SCF₂, ³*J*_{F-F} = 5,7 Hz), -109,84 (t, 2F, CF₂SO₂N, ³*J*_{F-F} = 5,7 Hz).
- **RMN** ¹**H**: (300 MHz, CDCl₃) : δ 4, 45 (d, 2H, H8, ³*J*_{H7-H8} = 5,8 Hz), 5,05 (t, 1H, NH, ³*J*_{H7-H8} = 5,8 Hz) 7,31-7,53 (m, 8H, H_{Ar}), 7,68 (d, 2H, H₃, ³*J*_{H2-H3} = 7,2 Hz).
- **RMN** ¹³**C**: (75 MHz, CDCl₃) : δ 48,52 (s, C8), 116,09 (tt, 1C, CF₂, ¹*J*_{F-C} = 294,2 Hz, ²*J*_{F-C} = 36,8 Hz), 123,24 (tt, 1C, CF₂, ¹*J*_{F-C} = 290,3 Hz, ²*J*_{F-C} = 32,3 Hz), 123,51 (t, 1C, C_{Ar4}, ³*J*_{F-C} = 2,7 Hz), 128, 00 (s, C_{Ar}), 128,56 (s, C_{Ar}), 129, 07 (s, C_{Ar}), 129, 48 (s, C_{Ar}), 131, 077 (s, C_{Ar}), 135, 67 (s, C_{Ar9}), 137, 45 (s, 2C, C_{Ar3}).

### Exemple 9

### N-Benzyl-2-(phénylsulfanyl)-1,1,2,2-tétrafluoro-N-(trifluorométhylsulfonyl)éthanesulfonamide

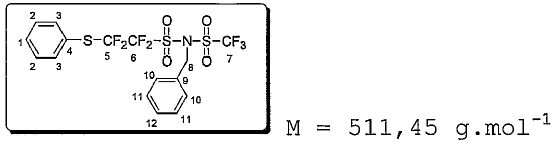

A une solution de dichlorométhane (10 mL) contenant le PhSCF₂CF₂SO₂NHBn préparé selon l'exemple 8 (750 mg, 2 mmol) on ajoute de la DIEA (420 µL, 2,4 mmol). De l'anhydride triflique (CF₃SO₂)₂O (510 µL, 3 mmol) est ensuite ajouté au mélange à 0°C qui est agité 30 min à cette température puis 1 h à température ambiante (suivi RMN ¹⁹F/CDCl₃). Une solution aqueuse de HCl (3%) est ajoutée au mélange, les phases aqueuses et organiques sont séparées et la phase aqueuse est extraite par du dichlorométhane. Les phases organiques sont regroupées et séchées sur MgSO₄ et les solvants sont évaporés. Le résidu est dissous dans du pentane à chaud et le surnageant est récupéré. Après évaporation du solvant, le sulfonamide est obtenu sous forme de solide blanc (925 mg, 91%).
- **RMN** ¹⁹**F**: (282 MHz, CDCl₃) : δ -72, 82 (s, 3F, CF₃), -85, 89 (s large, 2F, SCF₂), -102,85 (s large, 2F, CF₂SO₂N).
- **RMN** ¹**H**: (300 MHz, CDCl₃) : δ 5,07 (s, 2H, H8), 7,37-7,55 (m, 8H, H_{Ar}), 7,65 (d, 2H, H₃, ³*J*_{H2-H3} = 7,4 Hz).
- **RMN** ¹³**C**: (75 MHz, CDCl₃) : δ 56,56 (s, C8), 116,70 (tt, 1C, CF₂, ¹*J*_{F-C} = 305,2 Hz, ²*J*_{F-C} = 39,0 Hz), 119, 15 (q, 1C, C7, ¹*J*_{F-C} = 324, 9 Hz), 122,50 (tt, 1C, CF₂, ¹*J*_{F-C} = 292,4 Hz, ²*J*_{F-C} = 31,3 Hz), 122,89 (t, 1C, C_{Ar4,} ³*J*_{F-C} = 3,3 Hz), 128,89 (s, C_{Ar}), 129,65 (s, C_{Ar}), 129,71 (s, C_{Ar}), 130,12 (s, C_{Ar}), 131,43 (s, C_{Ar}), 132,27 (s, C_{Ar9}), 137,52 (s, 2C, C_{Ar3})

### Exemple 10

### 2-(phénylsulfanyl)-1,1,2,2-tétrafluoro-N-(trifluoro-méthylsulfonyl)éthanesulfonamidure de lithium

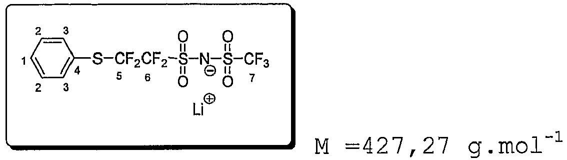

On prépare une solution dans l'éthanol (5 mL) du composé préparé selon l'exemple 9 (510 mg, 1 mmol). Le mélange est agité 8 h à température ambiante puis LiOH.H₂O (42 mg, 1 mmol) est ajouté. Le mélange est alors agité pendant une nuit puis évaporé à sec. Le résidu est dissous dans l'éther diéthylique puis filtré. Après évaporation du filtrat et lavage du solide résultant par du pentane, le sel de lithium est obtenu sous forme de solide blanc (385 mg, 90%).
- **RMN** ¹⁹**F**: (282 MHz, Acetone D6) : δ -80,29 (s, 3F, CF₃), -84, 94 (t, 2F, SCF₂, ³*J*_{F-F} = 5.7 Hz), -112,04 (t, 2F, CF₂SO₂N, ³*J*_{F-F} = 5,7 Hz).
- **RMN** ¹**H**: (300 MHz, Acetone D6) : δ 7, 46-7, 58 (m, 3H, H_{Ar}), 7, 67-7, 70 (m, 2H, H₃).
- **RMN** ¹³**C**: (75 MHz, Acetone D6) : δ 115,06 (tt, 1C, CF₂, ¹*J*_{F-C} = 293,1 Hz, ²*J*_{F-C} = 34,4 4 Hz), 120,59 (q, 1C, C7, ¹*J*_{F-C} = 321, 5 Hz), 123, 99 (tt, 1C, CF₂, ¹*J*_{F-C} = 290,4 Hz, ²*J*_{F-C} = 32,5 Hz), 124,41 (m, 1C, C_{Ar4}), 130,19 (s, C_{Ar}), 131, 59 (s, C_{Ar}), 137, 86 (s, 2C, C_{Ar3})

### Exemple 11

### 2-(phénylsulfanyl)-1,1,2,2-tétrafluoro-N-(trifluorométhanesulfonyl)éthanesulfonamidure de triéthylammonium

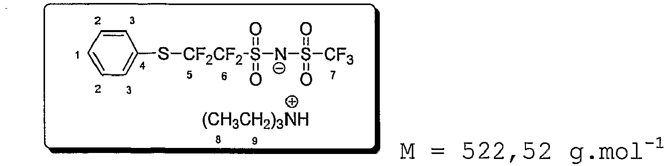

On prépare une solution dans l'éthanol (4 mL) du composé préparé selon l'exemple 9 (350 mg, 0,7 mmol). Le mélange est agité 8 h à température ambiante puis de la triéthylamine (100 µL, 0,7 mmol) est ajoutée. Le mélange est alors agité pendant une nuit puis évaporé à sec. Le résidu est lavé par du pentane (élimination du surnageant). Après séchage sous vide, le sel de triéthylammonium est obtenu sous forme de liquide jaune (300 mg, 82%).
- **RMN** ¹⁹**F**: (282 MHz, CDCl₃) : δ -79, 39 (s, 3F), -84, 97 (m, 2F, PhSCF₂), -110,65 (m, 2F, CF₂CF₂SO₂N).
- **RMN** ¹**H**: (300 MHz, CDCl₃) : δ 1,23 (t, 9H, H8, ³*J*_{H8-H9} = 7.3 Hz), 3,07-3,14 (m, 6H, H9), 6,78 (s large, 1H, NH), 7, 34-7, 47 (m, 3H, H_{Ar}), 7,62 (d, 2H, H3, ³*J*_{H2-H3} = 7,2 Hz).
- **RMN** ¹³**C**: (75 MHz, CDCl₃) : δ 114, 65 (tt, ¹*J*_{C-F} = 294, 2 Hz, ²*J*_{C-F} = 35,9 Hz), 119,72 (q, C7, ¹*J*_{C-F} = 320, 9 Hz), 123, 26 (tt, ¹*J*_{C-F} = 290, 0 Hz, ²*J*_{C-F} = 32, 0 Hz), 123,46 (t, C_{Ar4}, ³*J*_{C-F} = 2,7 Hz), 129,31 (s, C_{Ar2}), 130,90 (s, C_{Ar1}),137,24 (S, C_{Ar3}).

Le sel de triéthylammonium obtenu a été séché sous vide pendant 48 heures à 120°C pour éliminer toute trace d'eau, puis stocké en boîte à gants sous argon. Le liquide visqueux obtenu après ce traitement a été utilisé pour imprégner un séparateur macroporeux en polyéthylène/polypropylène pendant 12 heures en boîte à gants. Ensuite, on a monté le séparateur imprégéné dans une cellule de mesure de conductivité est alors montée et on a effectué les mesures de 25 à 110°C. Une conductivité de 1,8 mS/cm a été obtenue à 110°C. La mesure effectuée sur le sel pur, c'est-à-dire sans support macroporeux a fourni une valeur de 4,5 mS/cm à 110°C.

### Exemple 12

### 2-(phénylsulfanyl)-1,1,2,2-tétrafluoro-N-[2-(phényl-sulfanyl)-1,1,2,2-tétrafluoroéthanesulfonyl]-éthanesulfonamide de lithium

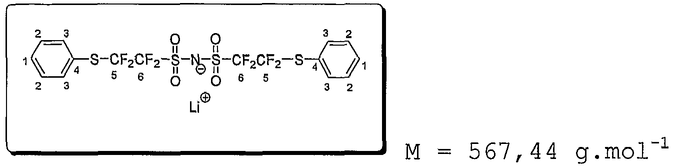

Une solution 1M (1 mL, 1mmol) du dérivé lithié de l'hexaméthyldisilazane dans le THF est ajouté à -20°C au composé PhSCF₂CF₂SO₂F (584 mg, 2 mmol), le mélange est agité à température ambiante pendant 24 h puis chauffé à 70°c pendant 6 jours. Le mélange est évaporé et le solide résiduel lavé au pentane. Le sel de lithium recherché est obtenu sous forme d'un solide blanc (230 mg, 40%) contaminé par quelques impuretés fluorées.
- **RMN** ¹⁹**F**: (282 MHz, CDCl₃) : δ -83,33 (t, 2F, SCF₂, ³*J*_{F-F} = 8,0 Hz), -112,78 (t, 2F, CF₂SO₂N, ³*J*_{F-F} = 8,0 Hz).
- **RMN** ¹**H**: (300 MHz, CDCl₃) : δ 7,43-7,55 (m, 3H, H₁ et H₂), 7,60-7,63 (m, 2H, H₃).

### Exemple 13

### N-Benzyl-2-(phénylsulfonyl)-1,1,2,2-tétrafluoroéthanesulfonamide

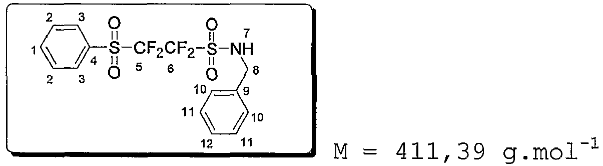

A une solution de 1,2-dichloroéthane (5 mL), contenant le PhSO₂CF₂CF₂SO₂F (325 mg, 1 mmol) préparé selon le procédé de l'exemple 4, on ajoute de la benzylamine fraichement distillée (550 µL, 5 mmol). Le mélange est agité et chauffé à 50°C pendant 20 h jusqu'à disparition de PhSO₂CF2CF₂SO₂F (suivi CCM et RMN ¹⁹F/CDCl₃). Après retour à température ambiante, une solution aqueuse de HCl (10%) est ajoutée au milieu réactionnel. Les phases aqueuses et organiques sont séparées et la phase aqueuse est extraite par du dichlorométhane. Les phases organiques sont regroupées et séchées sur MgSO₄. Après filtration et évaporation des solvants, le résidu est purifié par chromatographie sur gel de silice (Pentane à Pentane/AcOEt 4/1). Le sulfonamide est obtenu sous forme de solide blanc (340 mg, 83%).
- **RMN** ¹⁹**F**: (282 MHz, CDCl₃) : δ -110,09 (m, 2F), -110,25 (m, 2F).
- **RMN** ¹**H**: (300 MHz, CDCl₃) : δ 4,47 (d, 2H, H8, ³*J*_{H7-H8} = 5.7 Hz), 5,30 (t, 1H, NH, ³*J*_{H7-H8} = 5,7 Hz), 7,30-7,39 (m, 5H, H_{Ar}), 7,65-7,70 (m, 2H, H₂), 7,81-7,87 (m, 1H, H₁), 8,04 (d, 2H, H₃, ³*J*_{H2-H3} = 7,7 Hz).

### Exemple 14

### N-Benzyl-2-(phénylsulfonyl)-1,1,2,2-tétrafluoro-N-(trifluorométhanesulfonyl)éthanesulfonamide

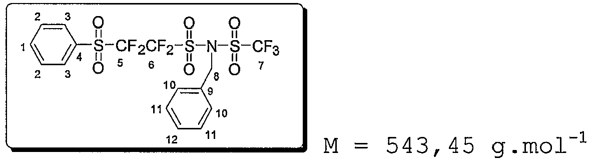

A une solution de dichlorométhane (5 mL), contenant le PhSO₂CF₂CF₂SO₂NHBn (205 mg, 0,5 mmol) obtenu selon le procédé de l'exemple 13, on ajoute de la DIEA (100 µL, 0,55 mmol). De l'anhydride triflique (150 µL, 0,85 mmol) est ensuite ajouté au mélange à 0°C qui est agité 30 min à cette température puis 1 h à température ambiante (suivi RMN ¹⁹F/CDCl₃). Une solution aqueuse de HCl (3%) est ajoutée au mélange, les phases aqueuses et organiques sont séparées et la phase aqueuse est extraite par du dichlorométhane. Les phases organiques sont regroupées et séchées sur MgSO₄ et les solvants sont évaporés. Le résidu est dissous dans du pentane à chaud et le surnageant est récupéré. Après évaporation du solvant, le sulfonimide est obtenu sous forme de solide blanc (120 mg, 44%).
- **RMN** ¹⁹**F**: (282 MHz, CDCl₃) : δ -72,67 (s, 3F, CF₃), -103,30 (s large, 2F), -110,23 (s large, 2F).
- **RMN** ¹**H**: (300 MHz, CDCl₃) : δ 5,07 (s, 2H, H₈), 7,26-7,49 (m, 5H, H_{Ar}), 7,65 (dd, 2H, H₂, ³*J*_{H1-H2} = ³*J*_{H2-H3} = 7,5 Hz), 7,85 (t, 1H, H₁, ³*J*_{H1-H2} = 7,5 Hz), 8,01 (d, 2H, H₃, ³*J*_{H2-H3} = 7,5 Hz).

### Exemple 15

### 2-(phénylsulfonyl)-1,2,2,2-tétrafluoro-N-(trifluorométhanesulfonyl)éthanesulfonamidure de lithium

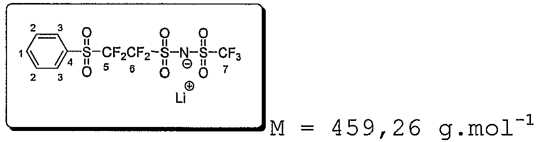

On prépare une solution dans l'éthanol (10 mL) du composé préparé selon l'exemple 14 (800 mg, 1,5 mmol). Le mélange est agité 8 h à température ambiante puis LiOH.H₂O (63 mg, 1 mmol) est ajouté. Le mélange est alors agité pendant une nuit puis évaporé à sec. Le résidu est dissous dans l'éther diéthylique puis filtré. Après évaporation du filtrat et lavage du solide résultant par du pentane, le sel de lithium est obtenu sous forme de solide blanc (400 mg, 59%).
- **RMN** ¹⁹**F**: (282 MHz, DMSO) : δ -79,17 (s, 3F, CF₃), -110,49 (m, 2F), -111,75 (m, 2F).
- **RMN** ¹**H**: (300 MHz, DMSO) : δ 7,78-7,84 (m, 2H, H_{Ar}), 7,96-8,02 (m, 1H, H_{Ar}), 8, 05-8, 08 (m, 2H, H_{Ar}).

### Exemple 16

### N-Benzyl-difluoro-(phénylsulfanyl)méthanesulfonamide

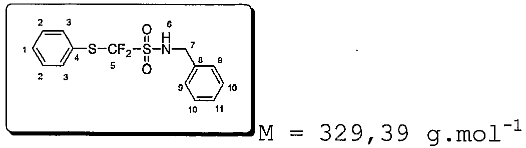

A une solution de 1,2-dichloroéthane (15 mL), contenant le PhSCF₂SO₂F (1,46 g, 5 mmol) obtenu selon le procédé de l'exemple 7, on ajoute de la benzylamine fraîchement distillée (2,7 mL, 25 mmol). Le mélange est agité et chauffé à 50°C pendant 20 h jusqu'à disparition de PhSCF₂SO₂F (suivi par CCM et RMN ¹⁹F/CDCl₃). Après retour à température ambiante, une solution aqueuse de HCl (10%) est ajoutée au milieu réactionnel. Les phases aqueuses et organiques sont séparées et la phase aqueuse est extraite par du dichlorométhane. Les phases organiques sont regroupées et séchées sur MgSO₄. Après filtration et évaporation des solvants, le résidu est purifié par chromatographie sur gel de silice (Pentane à Pentane/AcOEt 4/1). Le sulfonamide est obtenu sous forme de solide blanc (1,5 g, 90%).
- **RMN ¹⁹F**: (282 MHz, CDCl₃) : δ -77,99 (s, 2F)
- **RMN ¹H**: (300 MHz, CDCl₃) : δ 4, 40 (d, 2H, H7, ³*J*_{H6-H7} = 5,8 Hz), 4,94 (t, 1H, NH, ³*J*_{H6-H7} = 5,8 Hz), 7,29-7,53 (m, 8H, H_{Ar}), 7,70 (d, 2H, H3, ³*J*_{H2-H3} = 7,0 Hz).
- **RMN ¹³C**: (75 MHz, CDCl₃) : δ 48, 40 (s, C7), 123,77 (t, C_{Ar4}, ³*J*_{C-F} = 2.7 Hz), 127, 90 (s, C_{Ar}), 128, 07 (t, C5, ¹*J*_{C-F} = 320.0 Hz), 128,35 (s, C_{Ar}), 128, 95 (s, C_{Ar}), 129,46 (s, C_{Ar}), 131,01 (s, C_{Ar}), 136,06 (s, C_{Ar8}), 137,15 (t, C_{Ar3}, ⁴*J*_{C-F} = 1,1 Hz).

### Exemple 17

### N-Benzyl-difluoro-(phénylsulfanyl)-N-trifluorométhanesulfonylméthanesulfonamide

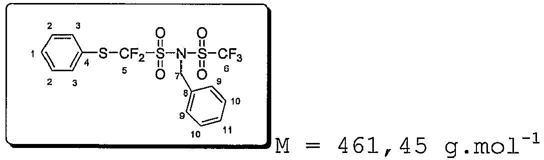

A une solution de dichlorométhane (10 mL), contenant le PhSCF₂SO₂NHBn préparé selon l'exemple 16 (1,49 g, 4,5 mmol) on ajoute de la DIEA (830 µL, 4,75 mmol). De l'anhydride triflique (CF₃SO₂)₂O (1,15 mL, 6,75 mmol) est ensuite ajouté au mélange à 0°C qui est agité 30 min à cette température puis 1 h à température ambiante (suivi RMN ¹⁹F/CDCl₃). Une solution aqueuse de HCl (3%) est ajoutée au mélange, les phases aqueuses et organiques sont séparées et la phase aqueuse est extraite par du dichlorométhane. Les phases organiques sont regroupées et séchées sur MgSO₄ et les solvants sont évaporés. Le résidu est dissous dans du pentane à chaud et le surnageant est récupéré. Après évaporation du solvant, le sulfonamide est obtenu sous forme de solide blanc (1,85 g, 89%).
- **RMN ¹⁹F**: (282 MHz, CDCl₃) : δ -72,59 (s, 2F), -72, 96 (s, 3F)
- **RMN ¹H**: (300 MHz, CDCl₃) : δ 5,03 (s, 2H, H7) 7,34-7,56 (m, 8H, H_{Ar}), 7,69 (d, 2H, H3, ³*J*_{H2-H3} = 7,3 Hz).
- **RMN ¹³C**: (75 MHz, CDCl₃) : δ 56,33 (s, C7), 118,99 (q, C6, ¹*J*_{C-F} = 324,9 Hz), 122,44 (t, C_{Ar4}, ³*J*_{C-F} = 3,0 Hz), 128,77 (s, C_{Ar}), 128,87 (t, C5, ¹*J*_{C-F} = 327,9 Hz), 129, 46 (s, C_{Ar}), 129,73 (s, C_{Ar}), 129,95 (s, C_{Ar}), 131,71 (s, C_{Ar}), 132,69 (s, C_{Ar8}), 137, 42 (t, C_{Ar3}, ⁴*J*_{C-F} = 1,1 Hz).

### Exemple 18

### difluoro-phénylsulfanyl-N-(trifluorométhanesulfonyl)méthanesulfonamidure de lithium

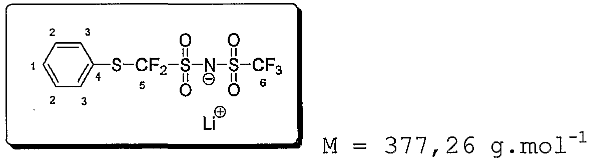

On prépare une solution dans l'éthanol (20 mL) du composé préparé selon l'exemple 17 (1,38 g, 3 mmol). Le mélange est agité 8 h à température ambiante puis LiOH.H₂O (125 mg, 3 mmol) est ajouté. Le mélange est alors agité pendant une nuit puis évaporé à sec. Le résidu est dissous dans l'éther diéthylique puis filtré. Après évaporation du filtrat et lavage du solide résultant par du pentane, le sel de lithium est obtenu sous forme de solide blanc (950 mg, 85%).
- **RMN ¹⁹F**: (282 MHz, Acetone D6) : δ -79,00 (s, 2F), -80,08 (s, 3F)
- **RMN ¹H**: (300 MHz, Acetone D6) : δ 7.41-7.53 (m, 3H, H_{Ar}), 7,63-7,66 (m, 2H, H3).
- **RMN ¹³C**: (75 MHz, Acetone D6) : δ 120,95 (q, C6, ¹*J*_{C-F} = 321,8 Hz), 126,05 (m, C_{Ar4}), 128,72 (t, C5, ¹*J*_{C-F} = 319,4 Hz), 130,01 (s, C_{Ar}), 131,12 (s, C_{Ar}), 137,37 (s, C_{Ar}).

### Exemple 19

### N-Benzyl-(phénylsulfonyl)difluorométhanesulfonamide

On a préparé le *N*-Benzyl-(phénylsulfonyl)difluorométhanesulfonamide à partir du fluorure de difluoro(phénylsulfonyl)méthanesulfonyle.

### Préparation du fluorure de difluoro(phénylsulfonyl)méthane-sulfonyle.

A une solution de PhSCF₂SO₂F (2,23 g, 9,2 mmol), préparé selon le mode opératoire décrit dans l'exemple 7, dans du dichlorométhane anhydre (90 mL) on a ajouté 12 g, (70 mmol) d'acide métachloroperbenzoïque. Le milieu réactionnel est agité à température ambiante pendant 3 jours (suivi par RMN ¹⁹F) puis filtré sur gel de silice et lavé par du CH₂Cl₂. Après évaporation du solvant, le résidu est purifié par chromatographie sur gel de silice (Pentane/CH₂Cl₂, 4/1). Le fluorure de difluoro(phénylsulfonyl)méthanesulfonyle est obtenu sous forme de liquide incolore.
- **CCM :**: *R_{f}* 0,65 (Pentane/CH₂Cl₂, 1/1)
- **RMN ¹⁹F**: (282 MHz, CDCl₃) : δ 49,28 (t, 1F, SO₂F, ³*J*_{F-F} = 5,7 Hz), -99,40 (d, 2F, CF₂, ³*J*_{F-F} = 5,7 Hz).
- **RMN ¹H**: (300 MHz, CDCl₃) : δ 7.70 (t 2H, H₂, ³*J*_{H2-H3} = ³*J*_{H2-H1} = 7,6 Hz), 7,90 (t, 1H, H₁, ³*J*_{H1-H2} = 7,6 Hz), 8,09 (d, 2H, H₃, ³*J*_{H2-H3} = 7,6 Hz).
- **RMN ¹³C**: (75 MHz, CDCl₃) : δ 118,76 (td, 1C, CF₂, ¹*J*_{F-C} = 336, 3 Hz, ³*J*_{F-C} = 30,0 Hz), 130,24 (s, 2C, C_{Ar 2}), 130, 99 (s, 1C, C_{Ar 4}), 131,58 (s, 2C, C _{Ar 3}), 137, 66 (s, 1C, C_{Ar 1}).

### Préparation du N-Benzyl-(phénylsulfonyl)difluorométhane-sulfonamide

On a mis en oeuvre le procédé suivant. A une solution du fluorure de sulfonyle (1,1 mmol) dans le 1,2-dichloroéthane anhydre (C ≈ 0,2 M) on a ajouté, sous atmosphère inerte et - 20 °C, de la benzylamine fraîchement distillée (5,5 mmol). Le mélange est agité et lentement ramené à température ambiante pendant 20 h jusqu'à disparition du fluorure de sulfonyle (suivi par CCM et RMN ¹⁹F/CDCl₃). Après retour à température ambiante, une solution aqueuse de HCl (10%) est ajoutée au milieu réactionnel. Les phases aqueuses et organiques sont séparées et la phase aqueuse est extraite par du dichlorométhane. Les phases organiques sont réunies et séchées sur MgSO₄. Après filtration et évaporation des solvants, le résidu est purifié par chromatographie sur gel de silice (Pentane à Pentane/AcOEt 4/1). On a ainsi obtenu 0,30 g (0,83 mmol) de N-benzyl sulfonamide à partir de 1,1 mmol de PhSO₂CF₂SO₂F (rendement : 78%) sous forme d'un solide blanc. 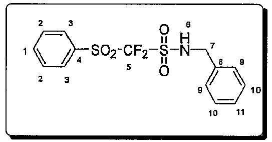
- M :: 361,38 g.mol⁻¹
- **CCM :**: *R_{f}* = 0,8 (pentane/AcOEt 4/1)
- **F.** :: 72°C
- **RMN ¹⁹F** :: δ = -102,36 (s)
- **RMN ¹H :**: δ = 4,49 (d, 2H, ³*J*_{H6-H7} = 5,8 Hz, H₇), 5,62 (t, 1H, ³*J*_{H6-H7} = 5,8 Hz, NH), 7, 34-7, 37 (m, 5H, H_{Ar}), 7,64 (m, 2H, H₂), 7,82 (m, 1H, H₁), 8,02 (m, 2H, H₃).
- **RMN ¹³C** :: δ = 48,39 (s, C₇), 118,91 (t, ¹*J*_{C-F} = 331, 2 Hz, C₅), 127,91 (s, C_{Ar}), 128,31 (s, C_{Ar}), 128,86 (s, C_{Ar}), 129, 62 (s, C_{Ar2}), 131,03 (m, C_{Ar3}), 132,11 (s, C_{Ar4} ou C_{Ar8}), 135, 68 (S, C_{Ar4} ou C_{Ar8}), 136, 50 (S, C_{Ar1}).

### Exemple 20

### N-Benzyl-N-trifluorométhanesulfonyl-(phénylsulfanyl)difluorométhanesulfonamide

On a mis en oeuvre le procédé suivant. A une solution ≈ 0,2 M dans le dichlorométhane de *N*-benzyl sulfonamide (1 éq.) obtenu selon le procédé de l'exemple 19, maintenue sous atmosphère inerte, on a ajouté de la DIEA (1,0 éq.). De l'anhydride triflique (1,5 éq.) est ensuite ajouté au mélange à 0°C. Ce mélange est agité pendant 30 min à cette température, puis pendant 1 h à température ambiante (suivi RMN ¹⁹F/CDCl₃). En fin de réaction, les produits volatils sont évaporés. Le résidu est dissous dans du pentane à chaud et le surnageant est récupéré. Après évaporation du solvant, on a obtenu 1,85 g (4,01 mmol) de *N*-benzyl sulfonimide qui répond à la formule ci-après, sous forme d'un solide blanc, à partir de 4,5 mmol de PhSCF₂SO₂NHBn (rendement : 89%). 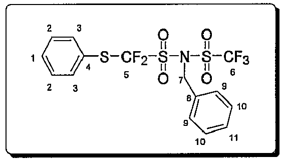
- M :: 461,45 g.mol-1
- F. :: 80-84°C
- **RMN ¹⁹F** :: δ = -72,59 (s, 2F), -72,96 (s, 3F)
- **RMN ¹H** :: δ = 5,03 (s, 2H, H₇) 7, 34-7, 56 (m, 8H, H_{Ar}), 7,69 (d, 2H, ³*J*_{H2-H3} = 7,3 Hz, H₃).
- **RMN ¹³C** :: δ = 56,33 (s, C₇), 118,99 (q, ¹*J*_{C-F} = 324,9 Hz, CF₃), 122,44 (t, ³*J*_{C-F} = 3,0 Hz, C_{Ar4}), 128,77 (s, C_{Ar}), 128,87 (t, ¹*J*_{C-F} = 327, 9 Hz, CF₂), 129,46 (s, C_{Ar}), 129,73 (s, C_{Ar}), 129,95 (s, C_{Ar}), 131,71 (s, C_{Ar}), 132,69 (s, C_{Ar8}), 137,42 (t, ⁴*J*_{C-F} = 1,1 Hz, C_{Ar3}).

### Exemple 21

### N-(trifluorométhanesulfonyl)-phénylsulfonyl-difluoro méthanesulfonamidure de lithium

On a mis en oeuvre le procédé suivant. Une solution de N-benzyl sulfonimide obtenu selon le procédé de l'exemple 20 (1 éq) dans l'éthanol (C ≈ 0,2 M) est agitée pendant 8 h à température ambiante, puis LiOH.H2O (1 éq.) est ajouté. Le mélange est alors agité pendant une nuit à température ambiante, puis évaporé à sec. Après évaporation des produits volatils, le résidu est dissous dans l'éther diéthylique puis filtré. Après évaporation du filtrat et lavage du solide résultant au pentane, on obtient 0,20 g (0,49 mmol) du sulfonimidure de lithium répondant à la formule ci-dessous, sous forme de solide blanc, à partir de 0,72 mml de PhSO₂CF₂SO₂N(Bn)SO₂CF₃ (rendement : 76%) 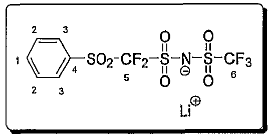
- M :: 409,26 g.mol⁻¹
- **RMN ¹⁹F :**: (Acétone-d₆) δ= -80,29 (s, 3F), -103,75 (s, 2F)
- **RMN ¹H** :: (Acétone-d₆) δ = 3, 14 (s large, H₂O liée), 7, 71 (m, 2H, H₂), 7,86 (m, 1H, H₁), 8,05 (m, 2H, H₃).
- **RMN ¹³C** :: (Acétone-d₆) δ= 119,46 (t, ¹*J*_{C-F} = 329,3 Hz, CF₂), 120,79 (q, ¹*J*_{C-F} = 321,3 Hz, CF₃), 130,12 (s, C_{Ar2}), 131,62 (s, C_{Ar3}), 134, 94 (s, C_{Ar4}), 136, 63 (s, C_{Ar1}).
- **SM :**: (**ESI**-MeOH) m/z = 402,0 (M⁻, 100%), 404,0 [(M+2)⁻, 10%], 810,6 [(2M+Li)⁻, 9%], 827,5 [(2M+Na)⁻, 7%].
- **SMHR :**: calculé pour C₈H₅F₅NO₆S₃: 401,9199; trouvé: 401,9193

### Exemple 22

### N-(trifluorométhanesulfonyl)-phénylsulfanyl-difluorométhanesulfonamidure de triéthylammonium

On a mis en oeuvre le procédé suivant. Une solution de N-benzyl sulfonimide (1 éq.) dans l'éthanol (5 mL) est agitée pendant 8 h à température ambiante puis de la tri-éthylamine (1 éq.) est ajoutée. Le mélange est agité pendant une nuit puis évaporé à sec. Après évaporation des produits volatils, le résidu est lavé par du pentane (élimination du surnageant). Après séchage sous vide, on a obtenu 0,37 g (0,79 mmol) du sel de triéthylammonium répondant à la formule ci-dessous, sous forme d'une huile jaune, à partir de 0,9 mmol de PhSCF₂SO₂N(Bn)SO₂CF₃. (rendement : 88%). 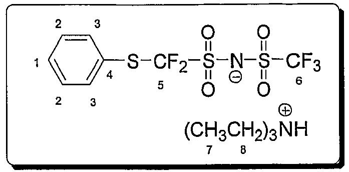
- **RMN ¹⁹F** :: δ = -78,62 (s, 2F), -79,09 (s, 3F)
- **RMN ¹H** :: δ = 1,33 (t, 9H, ³*J*_{H7-H8} = 7,3 Hz, H₇), 3,16 (q, 6H, ³*J*_{H7-H8} = 7,3 Hz, H₈), 7,08 (s large, NH), 7,35-7,48 (m, 3H, H_{Ar}), 7,69 (d, 2H, ³*J*_{H2-H3} = 7,3 Hz, H₃).
- **RMN ¹³C** :: δ = 8,51 (s, C₇), 47,01 (s, C₈), 119,77 (q, ¹*J*_{C-F} = 321,8 Hz, CF₃), 124,27 (t, ³*J*_{C-F} = 2,2 Hz, C_{Ar4}), 127,77 (t, ¹*J*_{C-F} = 319,7 Hz, CF₂), 129,26 (s, C_{Ar2}), 130, 64 (s, C_{Ar1}), 136, 86 (s, C_{A3r}).

### Exemple 23

### Préparation d'un film d'électrolyte polymère sec

On a préparé deux films d'électrolyte polymère sec à partir d'un poly(oxyde d'éthylène) POE (fourni par la société Aldrich, Mw=5,10⁶) et du 2-(phénylsulfanyl)-1,1,2,2-tétrafluoro-N-(trifluorométhylsulfonyl)-éthanesulfonamidure de lithium de l'exemple 10, séché sous vide dynamique pendant 48 h. POE et le sulfonimidure sont dissous dans de l'acétonitrile en boîte à gant sous argon. Deux essais ont été effectuées avec les proportions requises pour obtenir des rapport O/Li différents (rapport du nombre d'unité oxygène solvatant au nombre d'ions Li⁺ introduits). Chacune des solutions est laissée sous agitation pendant une nuit. Ensuite elle est coulée dans un anneau de verre collé sur une surface de téflon et laissées à évaporer une nuit. Les deux films dont l'épaisseur est entre 60 µm et 200 µm ont ensuite été séchés sous vide dynamique à 80°C pendant 48 h et stockés en boîte à gant.

Les électrolytes ont été caractérisés par calorimétrie différentielle à balayage DSC (differential scanning calorimetry) à 5°C par minute de -100°C à 100°C. Les capsules de mesures sont scellées en boîte à gant sous argon et conservées sous argon jusqu'au moment de la mesure où elles sont placées sous balayage d'azote, afin de prévenir toute prise d'eau qui perturberait les mesures. Chaque échantillon est soumis à une première montée en température suivie d'une descente rapide et d'une seconde montée en température. Les propriétés des polymères semi-cristallins dépendant en grande partie de l'histoire thermique du matériau, on s'assure ainsi d'une bonne reproductibilité des mesures au second passage. La température de transition vitreuse (Tg) est mesurée au point d'inflexion du thermogramme obtenu par DSC, et le point de fusion est déterminé au début du pic de fusion. Les résultats sont synthétisés dans le tableau I ci-dessous, dans lequel :
- O/li représente le rapport du nombre d'atomes d'oxygène solvatants au nombre d'atomes de lithium apportés par le sulfonylimidure dans le matériau obtenu par mélange de POE et du sulfonylimidure ;
- Tg₁ et Tg₂ représentent les températures de transition vitreuse déterminées respectivement au cours de la 1^{ère} et de la 2^{ème} montée en température ;
- Tf₁ et Tf₂ représentent les températures de fusion déterminées respectivement au cours de la 1^{ère} et de la 2^{ème} montée en température ;
- ΔH_{fus1} et ΔH_{fus2} représentent les enthalpies de fusion déterminées respectivement au cours de la 1^{ère} et de la 2^{ème} montée en température.

**Tableau I**

| O/Li | Tg₁(°C) | Tg₂(°C) | Tf₁(°C) | Tf₂(°C) | ΔH_{fus1}(J.g⁻¹) | ΔH_{fus2}(J.g⁻¹) |
|---|---|---|---|---|---|---|
| 23,2 | -30,5 | -31,6 | 48,9 | 48,6 | 73,7 | 72,8 |
| 13,9 | -30,5 | -32,7 | 35,6 | 36,6 | 43,4 | 38,5 |

Le sulfonylimidure de lithium diminue la cristallinité et la température de fusion des électrolytes polymères par rapport au POE pur. On en déduit que ce sel exerce un effet plastifiant.

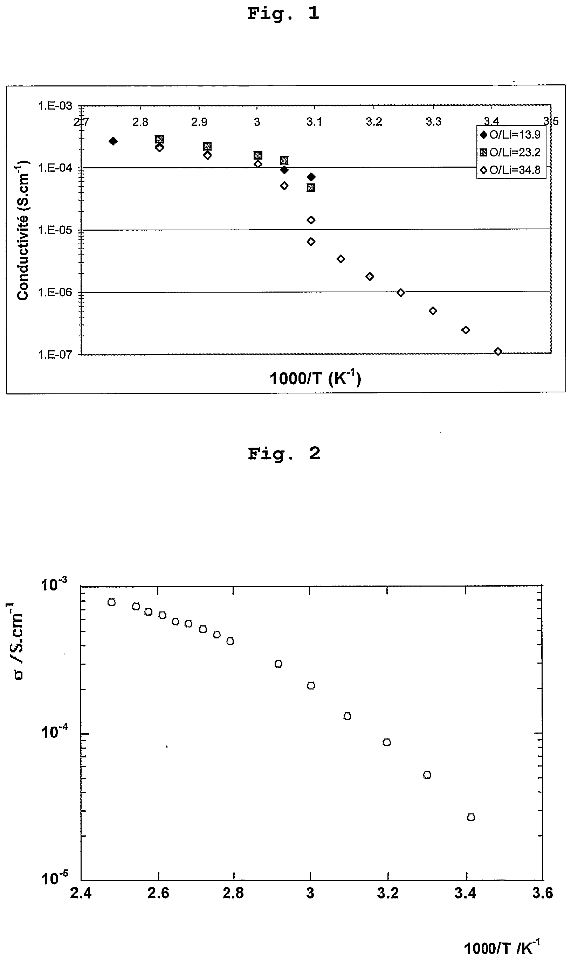

On a mesuré la conductivité d'électrolytes polymères secs POE/PhSCF₂SO₂NSO₂CF₃Li sur trois échantillons pour lequels le rapport O/LI était respectivement de 30,4, 23,2 et 13,9. Les mesures ont été réalisées par spectroscopie d'impédance électrochimique, en utilisant un spectromètre d'impédance HP 4192A LF impedance analyser. L'électrolyte a alors été monté en boîte à gant entre 2 cales en acier inoxydable dans une cellule Swagelok étanche, un ressort exerçant une pression constante sur l'électrolyte. Les mesures de conductivité ont été réalisées à des températures décroissantes jusqu'à 55°C après une stabilisation d'au moins 2 heures à une température de 80°C, avant le début des mesures, de manière à avoir un bon contact aux électrodes. Un temps de stabilisation d'au moins une heure est respecté pour chaque température. Après la mesure à 55°C, la cellule est refroidie jusqu'à 20°C. Une montée en température est alors réalisée après le maintien des cellules à 20°C pendant 12 h de manière à laisser le temps à une éventuelle cristallisation. Plusieurs mesures ont été effectuées pendant la stabilisation à 20°C. Les mesures de 20 à 50°C ont alors été effectuées à température croissante. La température est contrôlée au moyen d'une enceinte thermostatée Vôtsch Industrietechnik VTM 4004. L'épaisseur des films d'électrolyte est mesurée en fin de mesure, avec un palmer Mitutoyo IP 54, de manière à ce qu'une éventuelle diminution d'épaisseur due à un fluage à haute température résulte en une sous évaluation des conductivités à haute température. La figure 1 résume les résultats obtenus pour les électrolytes préparés comme expliqué ci-dessus avec le sel PhSCF₂SO₂NSO₂CF₃Li.

### Exemple 24

### (1-Phénylsulfanyl-difluororométhyl)trifluorométhyl sulfonylimidure d'hexylméthylimidazolium

A une solution de difluoro-phénylsulfanyl-N-(trifluoro-méthanesulfonyl)-méthanesulfonamidure de lithium préparé selon le procédé de l'exemple 18 (165 mg ; 0,44 mmole) dans l'acétone (6 ml), on ajoute le chlorure d'hexyl-méthylimidazolium (89 mg ; 0,44 mmole) solubilisé dans l'eau distillée (4 ml). La solution est agitée à température ambiante pendant 16 h, puis les solvants sont éliminés par évaporation sous vide. Le résidu est solubilisé dans le chloroforme (20 ml), puis lavé avec de l'eau distillée (20 ml). La phase aqueuse est extraite avec du chloroforme (2 * 20 ml) puis avec du dichlorométhane (3 * 20 ml). Les phases organiques sont réunies, séchées sur Na₂SO₄, et les solvants éliminés par évaporation sous vide pour donner le (1-Phénylsulfanyl-difluorométhyl)trifluorométhylsulfonylimidure d'hexylméthyl-imidazolium (186 mg/79%) sous forme d'huile jaune. La formule du composé est donnée ci-après.

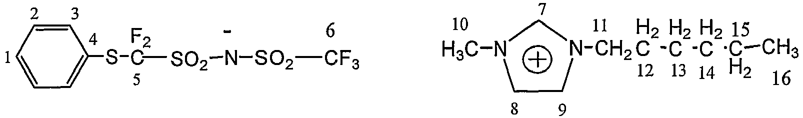

Les spectres RMN du proton ¹H, carbone ¹³C et du fluor ¹⁹F révèlent une réaction propre sans trace de matières premières résiduelles. Les déplacements chimiques des protons ¹H, du carbone ¹³C et du fluor ¹⁹F sont donnés dans les tableaux II, III et IV ci-dessous.
RMN 19F : δ = -78,60 (s, 2F), -79,30 (s, 3F)
RMN 1H . δ= 0,82 (m, 3H), 1,20-1,36 (m, 6H), 1,79 (m, 2H), 3,87 (m, 3H), 4,10 (m, 2H), 7,25-7,41 (m, 5H), 7,60-7,64 (m, 2H), 8,81 (m, 1H).

RMN 13C : δ = 13,80 (s), 22,26 (s), 25,71 (s), 30,02 (s), 30,91 (s), 36,27 (s), 50,06 (s), 122,07 (s, CAr), 123,63 (s, CAr), 124,62 (m, CAr4), 129,12 (s, CAr), 130,40 (s, CAr), 136,30 (s, CAr7), 136,88 (s, CAr). Le liquide ionique constitué par le sel d'imidazolium est séché 3 jours sous vide à 130°C pour éliminer toute trace d'eau, puis stocké en boîte à gants. Des mesures de conductivité sont effectuées sur un échantillon réalisé en imprégnant un séparateur en polyéthylène/polypropylène macroporeux par le liquide ionique. Le séparateur macroporeux est utilisé pour assurer la tenue mécanique. L'imprégnation a lieu durant 12 heures en boîte à gants, la cellule de conductivité est alors montée. Les mesures de conductivité sont effectuées de 20°C à 130°C avec une stabilisation de 2 h à chaque température. Les conductivités sont données sur la figure 2. La conductivité est de 4,7.10⁻⁴ S/cm à 90°C.

Les mesures de conductivité du sel d'imidazolium pur, sans membrane macroporeuse, ont également été réalisées, on obtient une conductivité de 6.5.10⁻⁴ à 90°C.

### Exemple 25

### Complexes copolymère réticulé /PhSCF₂CF₂SO₂NSO₂CF₃Li

On a préparé des électrolytes selon le mode opératoire suivant. Un macromonomère de masse moyenne 8000 g/mole ayant une fonction acrylate polymérisable à ses deux extrémités a été préparé à partir d'un mélange d'oxyde d'éthylène et d'oxyde de propylène. L'analyse RMN a montré que le macromonomère était un copolymère à tendance statistique comprenant 75 motifs oxyéthylène pour 25 motifs oxypropylène, ces derniers conférant au macromonomère un caractère amorphe.

3 g du macromonomère ont été dissous, en boîte à gants, dans l'acétonitrile. On y a ajouté du peroxyde de benzoyle de sorte à atteindre un rapport de 5% de peroxyde par rapport aux motifs acrylates. Après évaporation de l'acétonitrile à température ambiante pendant 12 heures en boîte à gants, le produit a été chauffé à 70°C pendant 1h30 en boite à gants, pour réaliser la réticulation.

On a ensuite découpé des disques dans la membrane obtenue après réticulation et on a extrait le polymère non réticulé à l'acétonitrile pendant 15 jours en renouvelant 6 fois le bain d'acétonitrile.

Les disques ont ensuite été traités sous vide pour éliminer l'acétonitrile et pesés. Une solution d'acétonitrile contenant la quantité de sel à incorporer est alors versée sur chaque disque. Les disques sont ensuite traités sous vide pendant 48 h afin d'éliminer l'acétonitrile, et stockés en boîte à gant. Les disques sont ensuite pesées pour déterminer la quantité de sel incorporée. On a ainsi obtenu des électrolytes en forme de disques, dans lesquels la concentration O/Li est respectivement de 14,0, 29,3 et 35,3.

### Caractérisation thermique

Les électrolytes ont été caractérisés par DSC (differential scanning calorimetry) à 5°C par minute de -100°C à 100°C. Les capsules de mesures sont scellées en boîte à gants sous argon et conservées sous argon jusqu'au moment de la mesure où elles sont placées sous balayage d'azote sec, afin d'empêcher toute prise d'eau qui perturberait les mesures. La température de transition vitreuse Tg est respectivement de -49°C, -47°C et -36°C pour les concentrations O/Li de 14,0, 29,3 et 35,3.

### Mesures de conductivité

Les mesures de conductivité ont été réalisées pour un complexe copolymère réticulé / PhSCF₂CF₂SO₂NSO₂CF₃Li ayant un taux O/Li de 35,3, par spectroscopie d'impédance électrochimique, en utilisant un spectromètre d'impédance HP 4192A LF impedance analyser. L'électrolyte a été monté en boîte à gants entre 2 cales en acier inoxydable dans une cellule Swagelok étanche. Un ressort exerçant une pression constante sur l'électrolyte. Les mesures de conductivité ont été réalisées à température décroissante de 90°C jusqu'à -10°C avec une stabilisation de 2 heures pour chaque température, avant le début des mesures. La température est contrôlée au moyen d'une enceinte thermostatée Vôtsch Industrietechnik VTM 4004. L'épaisseur des films d'électrolyte est mesurée avec un palmer Mitutoyo IP 54.

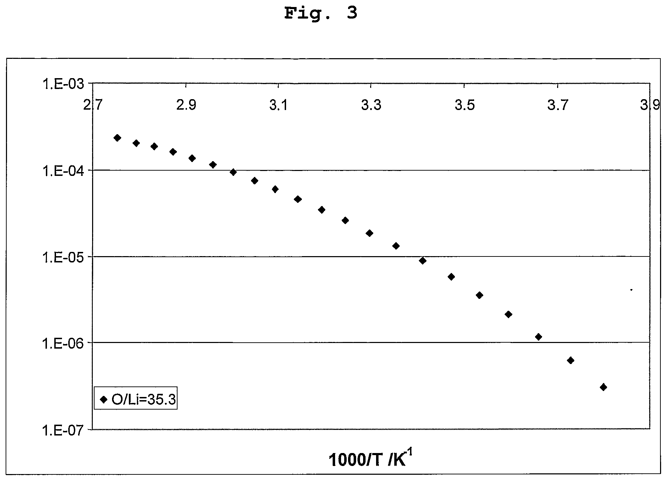

La figure 3 représente l'évolution de la conductivité (S.cm⁻¹), en fonction de la température. En raison du caractère amorphe, les conductivités en fonction de l'inverse de la température suivent un comportement VTF (Vogel-Tamman-Fulcher). Ainsi des conductivités de 0,01 mS/cm sont obtenues dès de 25°C et la conductivité dépasse 0,1 mS/cm au-dessus de 60°C.

## Revendications

1. Procédé de préparation d'un sulfonylimidure répondant à la formule [R-SO₂-N-SO₂R']ᵣM (I), **caractérisé en ce qu'**il consiste à préparer un sulfonimide N-substitué (II) RSO₂N(R")SO₂R' à partir de RSO₂F (III), et à remplacer le groupe R" par une réaction de substitution nucléophile pour obtenir le sulfonylimidure (I), R" étant un groupe benzyle, allyle ou triméthylsilyle, étant entendu que, dans les formule (I), (II) et (III) :
• R représente un groupe répondant à la formule Ar-Z-L- ;
• R' représente un groupe perfluoroalkyle linéaire ou ramifié, ou un groupe identique au groupe Ar-Z-L- qui constitue R ;
• Z représente un groupe sulfure, sulfinyle ou sulfonyle ;
• L représente un groupe de formule -(CF₂)ₙ-CFR^{f}- ;
• n est 0 ou 1 ;
• R^{f} représente F ou un groupe perfluoroalkyle ayant de 1 à 8 atomes de carbone ;
• Ar représente un groupement aromatique choisi dans le groupe constitué par les groupements aromatiques monocycliques ; les groupements aromatiques polycycliques ayant des noyaux condensés ou non condensés ; et les groupements aromatiques hétérocycliques, bicycliques à cycles condensés ou non, ou monocycliques ;
• M est un cation de valence r, choisi parmi les cations de métal alcalin, de métal alcalino-terreux, ou de métal trivalent ou tétravalent, et les cations organiques choisi parmi les ions ammonium, phosphonium, imidazolium, guanidinium, pipéridinium, pyrolidinium, pyridinium, ou quinolinium, et que
lorsque le groupement R' est un groupe identique au groupe Ar-Z-L- qui constitue R, le composé (II) répond à la formule (IIa) RSO₂N(SiMe₃)SO₂R, et il est obtenu par réaction du composé R-SO₂F (III) avec un sel d'hexaméthyldisilazane (CH₃)₃Si)₂N⁻A⁺ (A étant un cation de métal alcalin ou un ion ammonium quaternaire), et
lorsque le groupement R' est un groupe perfluoroalkyle, le composé (II) répond à la formule (IIb) RSO₂N(R")SO₂R', R" étant un groupe benzyle ou un groupe allyle, et il est obtenu par l'intermédiaire d'un composé RSO₂NH(R") (II'b) selon un procédé comprenant les étapes suivantes:
- réaction de R-SO₂F (III) avec la benzylamine ou l'allylamine en excès,
- neutralisation par HCl et l'isolement du composé R-SO₂NH(R") (II'b),
- réaction du composé (II'b) avec l'anhydride (R'SO₂)₂O, en présence d'une amine tertiaire, pour obtenir le composé (II) R-SO₂N(R")SO₂R'.

2. Procédé selon la revendication 1, pour la préparation d'un composé (I) dans lequel R' est un groupe CF₃ ou C₂F₅.

3. Procédé selon la revendication 1, **caractérisé en ce que** l'on fait réagir le composé (IIb) R-SO₂N(R")SO₂R' avec un réactif choisi parmi les hydroxydes et les halogénures de métal alcalin ou de métal alcalino-terreux, les hydroxydes et les halogénures d'ion ammonium quaternaire, les alcoolates de métal alcalin ou de métal alcalino-terreux, les alcoolates d'ammonium quaternaire, les amidures de métal alcalin ou de métal alcalino-terreux, les amidures d'ammonium quaternaire, les amines secondaires et les amines tertiaires.

4. Procédé selon la revendication 3, **caractérisé en ce que** la réaction est effectuée en présence d'un alcool.

5. Procédé selon la revendication 1, **caractérisé en ce que** M est un cation organique portant un ou plusieurs substituant(s) choisis indépendamment les uns des autres parmi :
- l'hydrogène ;
- les groupes alkyle ;
- les groupements aromatiques monocycliques ; les groupements aromatiques polycycliques à noyaux condensés ou non condensés ; les groupements aromatiques hétérocycliques dans lesquels l'hétéroatome est un atome d'azote, lesdits groupements hétérocycliques étant polycycliques à noyaux condensés ou non condensés, ou monocycliques.

6. Procédé selon la revendication 1, **caractérisé en ce que** Ar fait partie d'une unité récurrente d'une chaîne polymère.

7. Procédé selon la revendication 1, **caractérisé en ce que** le groupe Ar porte un ou plusieurs substituants choisis parmi :
• les atomes d'halogène, Cl-CH₂-, et les groupes Q¹-O-CH₂- dans lesquels Q¹ est H, un groupe alkyle ou un groupe acyle ;
• Un groupement hydroxyle, les groupements éther Q²-O-, ester carboxylique Q²C(O)O- et sulfonate Q²-SO₂-O-, Q² représentant un groupement alkyle ou un groupement aryle ;
• les groupements aliphatiques ou aromatiques possédant une insaturation éthylénique, substitué ou non ;
• les groupements amino -N(Q³)(Q⁴) - dans lesquels Q³ et Q⁴ représentent chacun indépendamment de l'autre H, un groupe alkyle, un groupe aryle, un groupe arylalkyle ou un groupe acyle ;
• les groupes trialkylsilyle ;
• les groupes oxirane ;
• les groupements électroattracteurs choisis parmi les groupements perfluoroalkyle, les groupes alkylsulfonyle ou arylsulfonyle, les groupes halogénure de sulfonyle, les groupes ester, nitrile, carbonate cyclique ou nitro.

## Patentansprüche

1. Verfahren zur Herstellung eines Sulfonylimids, das der Formel [R-SO₂-N-SO₂R¹]ᵣM (I) entspricht, **dadurch gekennzeichnet, dass** es darin besteht, ein N-substituiertes Sulfonimid (II) RSO₂N(R") SO₂R' ausgehend von RSO₂F (III) herzustellen und die Gruppe R" durch eine nucleophile Substitutionsreaktion zu ersetzen, um das Sulfonylimid (I) zu erhalten, wobei R" eine Benzyl-, Allyl- oder Trimethylsilylgruppe ist, wobei es sich versteht, dass in den Formeln (I), (II) und (III):
• R eine Gruppe darstellt, die der Formel Ar-Z-L- entspricht,
• R' eine lineare oder verzweigte Perfluoralkylgruppe oder eine Gruppe, die mit der Gruppe Ar-Z-L- identisch ist, darstellt,
• Z eine Sulfid-, Sulfinyl- oder Sulfonylgruppe darstellt,
• L eine Gruppe der Formel -(CF₂)ₙ-CFR^{f}- darstellt,
• n 0 oder 1 ist,
• R^{f} F oder eine Perfluoralkylgruppe mit 1 bis 8 Kohlenstoffatomen darstellt,
• Ar eine aromatische Gruppe darstellt, die aus der Gruppe ausgewählt ist, die von den monocyclischen aromatischen Gruppen, den polycyclischen aromatischen Gruppen mit kondensierten oder nicht kondensierten Kernen und den heterocyclischen, bicyclischen mit kondensierten oder nicht kondensierten Cyclen oder monocyclischen aromatischen Gruppen gebildet ist,
• M ein Kation mit der Wertigkeit r ist, ausgewählt aus den alkalischen, erdalkalischen oder drei- oder vierwertigen Metallionen und den organischen Kationen, ausgewählt aus den Ammonium-, Phosphonium-, Imidazolium-, Guanidinium-, Piperidinium-, Pyrolidinium-, Pyridinium- oder Quinoliniumionn, und dass
wenn die Gruppe R' eine Gruppe ist, die mit der Gruppe Ar-Z-L- identisch ist, die R bildet, die Verbindung (II) der Formel (IIa) RSO₂N(SiMe₃)SO₂R entspricht, und sie durch Reaktion der Verbindung R-SO₂F (III) mit einem Hexamethyldisilazansalz (CH₃)₃Si)₂N⁻A⁺ erhalten wird (wobei A ein kationisches Metallion oder ein quaternäres Ammoniumion ist), und
wenn die Gruppe R' eine Perfluoralkylgruppe ist, die Verbindung (II) der Formel (IIb) RSO₂N(R")SO₂R' entspricht, wobei R" eine Benzylgruppe oder eine Allylgruppe ist, und sie über eine Verbindung RSO₂NH(R") (II'b) gemäß einem Verfahren erhalten wird, das die folgenden Schritte umfasst:
- Reaktion von R-SO₂F (III) mit dem überschüssigen Benzylamin oder Allylamin,
- Neutralisierung durch HCl und Isolierung der Verbindung R-SO₂NH(R") (II'b),
- Reaktion der Verbindung (II'b) mit dem Anhydrid (R'SO₂)₂O bei Anwesenheit eines tertiären Amins, um die Verbindung (II) R-SO₂N(R")SO₂R' zu erhalten.

2. Verfahren nach Anspruch 1 für die Herstellung einer Verbindung (I), wobei R' eine Gruppe CF₃ oder C₂F₅ ist.

3. Verfahren nach Anspruch 1, **dadurch gekennzeichnet, dass** man die Verbindung (IIb) RSO₂N(R")SO₂R' mit einem Reagenz in Reaktion versetzt, das aus den alkalischen oder erdalkalischen Metallhydroxiden und -halogeniden, den Hydroxiden und den Halogeniden quaternärer Ammoniumionen, den alkalischen oder erdalkalischen Metalllalcoolaten, den quaternären Ammoniumalcoolaten, den alkalischen oder erdalkalischen Metallamiden, den quaternären Ammoniumamiden, den sekundären Aminen und den tertiären Aminen ausgewählt ist.

4. Verfahren nach Anspruch 3, **dadurch gekennzeichnet, dass** die Reaktion bei Anwesenheit eines Alkohols durchgeführt wird.

5. Verfahren nach Anspruch 1, **dadurch gekennzeichnet, dass** M ein organisches Kation ist, das einen oder mehrere Substituenten trägt, die unabhängig voneinander ausgewählt sind aus:
- dem Wasserstoff,
- den Alkylgruppen
- den monocyclischen aromatischen Gruppen, den polycyclischen aromatischen Gruppen mit kondensierten oder nicht kondensierten Kernen, den heterocyclischen aromatischen Gruppen, in welchen das Heteroatom ein Stickstoffatom ist, wobei die heterocyclischen Gruppen polycyclisch mit kondensierten oder nicht kondensierten Kernen oder monocyclisch sind.

6. Verfahren nach Anspruch 1, **dadurch gekennzeichnet, dass** Ar Teil einer rekurrenten Einheit einer Polymerkette ist.

7. Verfahren nach Anspruch 1, **dadurch gekennzeichnet, dass** die Gruppe Ar einen oder mehrere Substituenten trägt, die ausgewählt sind aus:
• den Halogenatomen, Cl-CH₂- und den Gruppen Q¹-O-CH₂-, in welchen Q¹ H, eine Alkylgruppe oder eine Acylgruppe ist,
• einer Hydroxylgruppe, den Ether- Q²-O-, Carboxylester- Q²C(O)O- und Sulfonatgruppen Q²-SO₂-O-, wobei Q² eine Alkylgruppe oder eine Arylgruppe darstellt,
• den aliphatischen oder aromatischen Gruppen, die eine substituierte oder nicht substituierte Etylenunsättigung besitzen,
• den Amingruppen -N(Q³)(Q⁴)-, in welchen Q³ und Q⁴ jeweils unabhängig voneinander H, eine Akylgruppe, eine Arylgruppe, eine Arylalkylgruppe oder eine Acylgruppe darstellen,
• den Trialkylsilylgruppen,
• den Oxirangruppen,
• den elektroattraktiven Gruppen, ausgewählt aus den Perfluoralkylgruppen, den Alkylsulfonyl- oder Arylsulfonylgruppen, den Sulfonylhalogenidgruppen, den Ester-, Nitril-, cyclischen Carbonat- oder Nitrogruppen.

## Claims

1. Method for preparing a sulfonylimide meeting the formula [R-SO₂-N-SO₂R'ᵣM (I), **characterized in that** it consists of preparing an N-substituted sulfonimide (II) RSO₂N(R")SO₂R' from RSO₂F (III), and of replacing the R" group via nucleophilic substitution reaction to obtain the sulfonylimide (I), R" being a benzyl, allyl or trimethylsilyl group, on the understanding that in formulas (I), (II) and (III):
• R is a group meeting the formula Ar-Z-L-;
• R' is a straight-chain or branched perfluoroalkyl group, or a group identical to the Ar-Z-L- group forming R;
• Z is a sulfide, sulfinyl or sulfonyl group;
• L is a group of formula -(CF₂)ₙ-CFR^{f}-;
• n is 0 or 1;
• R^{f} is F or perfluoroalkyl group having 1 to 8 carbon atoms;
• Ar is an aromatic group selected from the group composed of monocyclic aromatic groups; polycyclic aromatic groups having condensed or non-condensed cores; and heterocyclic, bicyclic aromatic groups with condensed or non-condensed rings, or monocyclic;
• M is a cation of valence r, selected from among cations of alkaline metals, alkaline-earth metals, or trivalent or tetravalent metals, and organic cations selected from among ammonium, phosphonium, imidazolium, guanidinium, piperidinium, pyrrolidinium, pyridinium or quinolinium ions, and **in that**:
when the R' group is a group identical to the Ar-Z-L- group forming R, compound (II) meets the formula (IIa) RSO₂N(SiMe₃)SO₂R, and it is obtained via reaction of compound R-SO₂F (III) with a hexamethyldisilazane salt (CH₃)₃Si)₂N^{-A+} (A being an alkaline metal cation or a quaternary ammonium ion), and
when group R' is a perfluoroalkyl group, the compound (II) meets the formula (IIb) RSO₂N(R")SO₂R', R" being a benzyl group or allyl group, and it is obtained via a compound RSO₂NH(R") (II'b) with a method comprising the following steps:
- reaction of R-SO₂F (III) with excess benzylamine or allylamine,
- neutralisation with HCl and isolation of the compound R-SO₂NH(R") (II'b);
- reaction of compound (II'b) with the anhydride (R'SO₂)₂O, in the presence of a tertiary amine, to obtain compound (II) R-SO₂N(R")SO₂R'.

2. The method according to claim 1, to prepare compound (I) wherein R' is a group CF₃ or C₂F₅.

3. The method according to claim 1, **characterized in that** compound (IIb) R-SO₂N(R")SO₂R' is caused to react with a reagent selected from among hydroxides and halides of an alkaline metal or alkaline-earth metal, hydroxides and halides of a quaternary ammonium ion, alcoholates of an alkaline or alkaline-earth metal, quaternary ammonium alcoholates, amides of an alkaline or alkaline-earth metal, quaternary ammonium amides, secondary amines and tertiary amines.

4. The method according to claim 3, **characterized in that** the reaction is conducted in the presence of an alcohol.

5. The method according to claim 1, **characterized in that** M is an organic cation carrying one or more substituents each independently selected from among:
- hydrogen;
- alkyl groups;
- monocyclic aromatic groups; polycyclic aromatic groups with condensed or non-condensed cores; heterocyclic aromatic groups where the heteroatom is a nitrogen atom, said heterocyclic groups being polycyclic with condensed or non-condensed cores, or monocyclic.

6. The method according to claim 1, **characterized in that** Ar belongs to a recurrent unit of a polymer chain.

7. The method according to claim 1, **characterized in that** the Ar group carries one or more substituents selected from among:
• halogen atoms, Cl-CH₂-, and Q¹-O-CH₂- groups where Q¹ is H, an alkyl group or acyl group;
• a hydroxyl group, Q²-O- ether groups, carboxylic Q²C(O)O and sulfonate Q²-SO₂-O- ester, Q² being an alkyl group or aryl group;
• aliphatic or aromatic groups having ethylenic unsaturation, substituted or unsubstituted;
• amino groups -N(Q³)(Q⁴)- where Q³ and Q⁴ are each independently H, an alkyl group, aryl group, arylalkyl group or acyl group;
• trialkylsilyl groups;
• oxirane groups;
• electron attractor groups selected from among perfluoroalkyl groups, alkylsulfonyl or arylsulfonyl groups, sulfonyl halide groups, ester, nitrile, cyclic carbonate or nitro groups.
